(19) Europäisches Patentamt
European Patent Office
Office européen des brevets

(11) **EP 2 096 989 B1**

(12) **EUROPÄISCHE PATENTSCHRIFT**

(45) Veröffentlichungstag und Bekanntmachung des Hinweises auf die Patenterteilung:
**21.11.2012 Patentblatt 2012/47**

(21) Anmeldenummer: **07856240.2**

(22) Anmeldetag: **23.11.2007**

(51) Int Cl.:
*A61B 5/00* (2006.01)        *A61B 5/02* (2006.01)
*A61B 5/022* (2006.01)        *A61B 5/024* (2006.01)
*A61B 5/0402* (2006.01)        *A61B 5/0404* (2006.01)
*A61B 5/01* (2006.01)        *A61B 5/1455* (2006.01)
*A61B 5/145* (2006.01)        *A61B 5/053* (2006.01)

(86) Internationale Anmeldenummer:
**PCT/EP2007/010207**

(87) Internationale Veröffentlichungsnummer:
**WO 2008/061788 (29.05.2008 Gazette 2008/22)**

(54) **MEDIZINISCHE MESSVORRICHTUNG**

MEDICAL MEASURING DEVICE

DISPOSITIF DE MESURE MÉDICAL

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **23.11.2006 DE 102006055691**
**07.09.2007 DE 102007042551**
**07.09.2007 DE 102007042550**

(43) Veröffentlichungstag der Anmeldung:
**09.09.2009 Patentblatt 2009/37**

(73) Patentinhaber: **Flore, Ingo**
**44141 Dortmund (DE)**

(72) Erfinder:
• **CHO, Ok Kyung**
**58239 Schwerte (DE)**

• **KIM, Yoon Ok**
**58239 Schwerte (DE)**

(74) Vertreter: **Isfort, Olaf**
**Schneiders & Behrendt**
**Rechtsanwälte - Patentanwälte**
**Huestrasse 23**
**44787 Bochum (DE)**

(56) Entgegenhaltungen:
**EP-A- 1 407 713        WO-A-01/65810**
**WO-A-96/01585        WO-A-2006/099988**
**WO-A2-2007/017266        DE-A1- 19 519 125**
**DE-U1- 29 811 049        DE-U1-202005 001 894**
**US-A1- 2003 036 685        US-A1- 2005 020 936**
**US-A1- 2005 078 533**

EP 2 096 989 B1

**Beschreibung**

Die Erfindung betrifft eine Messvorrichtung zur nicht-invasiven Bestimmung von wenigstens einem physiologischen Parameter, mit wenigstens einer diagnostischen Sensoreinheit zur Erzeugung von Messsignalen, und mit einer Auswertungseinheit zur Verarbeitung der Messsignale.

Die Versorgung des Körpergewebes mit Sauerstoff gehört bekanntlich zu den wichtigsten Vitalfunktionen des Menschen. Aus diesem Grund sind oximetrische Diagnosemodalitäten heutzutage von großer Bedeutung in der Medizin. Routinemäßig werden so genannte Pulsoximeter eingesetzt. Die diagnostische Sensoreinheit derartiger Pulsoximeter umfasst typischerweise zwei Lichtquellen, die rotes bzw. infrarotes Licht unterschiedlicher Wellenlänge in das Körpergewebe einstrahlen. Das Licht wird im Körpergewebe gestreut und teilweise absorbiert. Das gestreute Licht wird schließlich mittels eines Lichtsensors in Form einer geeigneten Photozelle (Photodiode) detektiert. Typischerweise verwenden kommerzielle Pulsoximeter zum einen Licht im Wellenlängenbereich von 660 nm. In diesem Bereich ist die Lichtabsorption von Oxihämoglobin und Desoxihämoglobin stark unterschiedlich. Dementsprechend variiert die Intensität des mittels des Photosensors detektierten, gestreuten Lichts in Abhängigkeit davon, wie stark das untersuchte Körpergewebe von sauerstoffreichem, bzw. sauerstoffarmem Blut durchblutet ist. Zum anderen wird üblicherweise Licht im Wellenlängenbereich von 810 nm verwendet. Diese Lichtwellenlänge liegt im so genannten nahen infraroten Spektralbereich. Die Lichtabsorption von Oxihämoglobin und Desoxihämoglobin ist in diesem Spektralbereich im Wesentlichen gleich. Die bekannten Pulsoximeter sind außerdem in der Lage, ein plethysmographisches Signal, d. h. ein Volumenpulssignal zu erzeugen, das die während des Herzschlags veränderliche Blutmenge in dem von dem Pulsoximeter erfassten Mikrogefäßsystem wiedergibt (sog. Photoplethysmographie). Bei Verwendung unterschiedlicher Lichtwellenlängen in den oben erwähnten Spektralbereichen kann aus der unterschiedlichen Lichtabsorption auf den Sauerstoffgehalt des Blutes (Sauerstoffsättigung) zurückgeschlossen werden. Die üblichen Pulsoximeter werden entweder an der Fingerspitze eines Patienten oder auch am Ohrläppchen eingesetzt. Es wird dann das Volumenpulssignal aus der Blutperfusion des Mikrogefäßsystems in diesen Bereichen des Körpergewebes erzeugt.

Aus der WO 00/69328 A1 ist ein besonders flexibel einsetzbares oximetrisches Diagnosegerät bekannt. Dieses vorbekannte Gerät ist handführbar ausgebildet, so dass es an beliebigen Messorten am menschlichen Körper eingesetzt werden kann. Das vorbekannte Gerät erlaubt gleichsam ein systematisches Abtasten ("Scannen") des Körpers eines Patienten. Eine Fixierung des Diagnosegerätes - wie bei üblichen Pulsoximetern - kann bei dem aus der genannten Druckschrift bekannten Gerät entfallen.

Die genannte WO 00/69328 A1 spricht außerdem die Einsetzbarkeit des oximetrischen Diagnosegerätes zur ortsaufgelösten Erkennung von Entzündungen, Tumoren und Arterioskleroseerkrankungen im hautoberflächennahen Körpergewebe eines Patienten an. Derartige Erkrankungen bewirken eine Veränderung der Durchblutung des Körpergewebes. Durch die ortsaufgelöste oximetrische Abtastung des Körpers lassen sich mit dem vorbekannten Gerät Veränderungen der Durchblutung, die auf eine entsprechende Erkrankung hindeuten, erkennen und lokalisieren.

Das EKG (Elektrokardiogramm) dürfte die am meisten eingesetzte Untersuchungsmodalität zur Diagnose von Herz-Kreislauf-Erkrankungen sein. Mittels der diagnostischen Sensoreinheit eines EKG-Gerätes werden mit zwei oder mehr EKG-Elektroden elektrische Signale von dem Körper des zu untersuchenden Patienten abgeleitet. Das so gewonnene EKG gibt die bioelektrischen Spannungen, die bei der Erregungsausbreitung und -rückbildung am Herzen entstehen, wieder. Das EKG enthält zahlreiche diagnostisch auswertbare Parameter. Zum Zeitpunkt der Kontraktion des Herzmuskels während eines Herzschlags zeigt das EKG eine deutliche Spitze, die auch als R-Zacke bezeichnet wird. Weiterhin enthält das EKG die der R-Zacke vorangehende, so genannte P-Welle. Der R-Zacke folgt wiederum die so genannte T-Welle. Die Minima im EKG unmittelbar vor und unmittelbar nach der R-Zacke werden mit Q bzw. S bezeichnet. Für die Herz-Kreislauf-Diagnostik interessante Parameter sind die Dauer der P-Welle sowie die Amplitude der P-Welle, die Dauer des PQ-Intervalls, die Dauer des QRS-Komplexes, die Dauer des QT-Intervalls sowie die Amplitude der T-Welle. Sowohl aus den Absolutwerten der genannten Parameter wie auch aus den Verhältnissen der Parameter kann auf den Gesundheitszustand des Herz-Kreislauf-Systems geschlossen werden. Vorrichtungen und Verfahren zur EKG-Messung sind beispielsweise aus den Druckschriften US 6,331,162 oder US 4,960,126 vorbekannt.

Zur Bestimmung von weiteren physiologischen Parametern, wie z. B. Körperfettgehalt, ist das Prinzip der bioelektrischen Impedanzmessung beispielsweise aus der US 6,714,814 bekannt. Die Zusammensetzung des Körpergewebes kann aber auch optisch bestimmt werden. Das Prinzip der optischen Bestimmung des Körperfettgehalts mittels Infrarotlicht ist beispielsweise in der US 4,928,014 beschrieben.

Der vorliegenden Erfindung liegt die Aufgabe zugrunde, eine Vorrichtung zur nicht-invasiven Bestimmung von physiologischen Parametern bereit zu stellen, die gegenüber dem Stand der Technik hinsichtlich ihrer Funktionalität erweitert ist. Insbesondere soll ein Gerät geschaffen werden, das vom Benutzer komfortabel und häufig verwendet werden kann, um eine zuverlässige und frühzeitige Erkennung von Erkrankungen sowie eine kontinuierliche Überwachung bestehender Erkrankungen zu ermöglichen. Aus EP- 1407713 ist ein mobiles Gerät der Unterhaltungs- oder Kommunikationstechnik mit einer diagnostischen Sensoreinheit bekannt. Diese Aufgabe löst die Erfindung ausgehend von einer Messvorrichtung gemäß Ansprüchen 1 und 2.

Durch die Integration der diagnostischen Sensoreinheit der Messvorrichtung in die Tastatur eines Computers oder in ein mobiles Gerät der Unterhaltungs- oder Kommunikationstechnik kann der Benutzer des Computers bzw. des mobilen Gerätes jederzeit die Messvorrichtung einsetzen, um die ihn interessierenden physiologischen Parameter zu bestimmen. Die meisten Personen haben heutzutage Zugang zu Computern, sei es im Büro oder zu Hause. Außerdem benutzen die meisten Menschen ein mobiles Kommunikationsgerät, wie z.B. ein Mobiltelefon oder ein so genanntes Smart Phone. Ebenfalls üblich ist die Benutzung von mobilen Unterhaltungsgeräten, wie z.B. MP3-Playern oder so genannten Walkmen. Die Integration der diagnostischen Messeinheit in die Tastatur des Computers oder in ein mobiles Gerät der zuvor angesprochenen Art stellt somit sicher, dass die Messvorrichtung vom Benutzer häufig verwendet werden kann. Der Benutzer kann praktisch gleichzeitig mit der Arbeit an dem Computer oder mit der Benutzung des entsprechenden Gerätes eine Messung zur Bestimmung der physiologischen Parameter durchführen. Vorteilhafterweise muss kein gesondertes Gerät hierfür verwendet werden. Vorteilhaft ist insbesondere auch, dass die Ermittlung von physiologischen Parametern unauffällig erfolgen kann, da sich die diagnostische Messung aus Sicht eines Dritten nicht von einer anderweitigen normalen Bedienung der Tastatur des Computers oder des mobilen Gerätes unterscheiden lässt. Besonders für Diabetiker bietet sich die erfindungsgemäße Messvorrichtung zur Bestimmung des Blutglukosespiegels an. Die erfindungsgemäße Messvorrichtung eignet sich weiterhin zur Überwachung und zur Dokumentation eines Therapieverlaufs bei einer vorliegenden Erkrankung. Der Benutzer kann regelmäßig die Messvorrichtung benutzen, um die für die Therapiekontrolle interessierenden Parameter zu erfassen. Diese können sodann (mit Zeit und Datum der Messung) protokolliert, gespeichert oder über ein geeignetes Datenübertragungsnetz übertragen werden.

Die diagnostische Sensoreinheit kann gemäß der Erfindung eine optische Messeinheit zur Erzeugung von oximetrischen und/oder plethysmographischen Messsignalen umfassen. Dies ermöglicht es, die Versorgung des Körpergewebes des Benutzers der Vorrichtung mit Sauerstoff zu überwachen und/oder ein Volumenpulssignal zu erzeugen.

Zweckmäßigerweise ist die Auswertungseinheit der erfindungsgemäßen Messvorrichtung eingerichtet zur Bestimmung wenigstens eines lokalen metabolischen Parameters, insbesondere des lokalen Sauerstoffverbrauchs, aus den Signalen der optischen Messeinheit. Die Auswertungseinheit zieht die mittels der optischen Messeinheit gewonnenen oximetrischen und/oder plethysmographischen Messsignale heran, um nicht nur die lokale Sauerstoffkonzentration am jeweiligen Messort, d. h. insbesondere an den Fingerspitzen des Benutzers der Tastatur oder des mobilen Gerätes, sondern insbesondere auch den lokalen Sauerstoffverbrauch als wichtigen Indikator für die lokale metabolische Aktivität zu bestimmen. Erkrankungen können mit der erfindungsgemäßen Messvorrichtung anhand von pathologischen Veränderungen des Metabolismus erkannt werden.

Die optische Messeinheit der erfindungsgemäßen Messvorrichtung weist eine Strahlungsquelle zur Bestrahlung des untersuchten Körpergewebes mit elektromagnetischer Strahlung, und wenigstens einen Strahlungssensor zur Detektion der von dem Körpergewebe gestreuten und/oder transmittierten Strahlung auf. Als Strahlungsquelle kommen übliche Leuchtdioden oder auch Laserdioden in Frage, die optische Strahlung, d.h. Licht im entsprechenden Spektralbereich emittieren. Als besonders vorteilhaft hat es sich erwiesen, wenn mit der erfindungsgemäßen Vorrichtung die Strahlungsabsorption im untersuchten Körpergewebe bei mindestens zwei oder besser drei unterschiedlichen Lichtwellenlängen gemessen wird, um daraus die Sauerstoffkonzentration des Blutes und die Durchblutung des Gewebes zu bestimmen.

Gemäß einer sinnvollen Ausgestaltung weist die optische Messeinheit der erfindungsgemäßen Messvorrichtung wenigstens zwei Strahlungssensoren zur Detektion der von dem Körpergewebe gestreuten und/oder transmittierten Strahlung auf, wobei die Strahlungssensoren in unterschiedlichem Abstand zur Strahlungsquelle angeordnet sind. Dies eröffnet die Möglichkeit, Rückschlüsse auf die jeweils im Körpergewebe von der Strahlung zurückgelegte Strecke zu ziehen. Auf dieser Basis kann die Sauerstoffkonzentration im Blut und im Gewebe in unterschiedlich tiefen Gewebeschichten untersucht werden. Dabei kann ausgenutzt werden, dass die Messsignale aus den tiefer liegenden Gewebeschichten stärker vom arteriellen Blut beeinflusst sind, während in den oberflächennäheren Regionen die Strahlungsabsorption stärker von dem Blut im kapillaren Gefäßsystem beeinflusst ist.

Vorteilhaft ist eine Ausgestaltung der erfindungsgemäßen Messvorrichtung, bei welcher wenigstens zwei Strahlungsquellen vorgesehen sind, welche unterschiedliche Volumenbereiche des untersuchten Körpergewebes bestrahlen. Hierdurch lässt sich eine differenzielle Messung der Lichtabsorption einfach realisieren. Dies ermöglicht es, Metabolismus-induzierte Änderungen der Durchblutung des untersuchten Körpergewebes mit sauerstoffreichem bzw. sauerstoffarmem Blut zu untersuchen. Dabei wird ausgenutzt, dass sich in Abhängigkeit von der metabolischen Aktivität des Gewebes der lokale Sauerstoffverbrauch verändert. Die Bestimmung des veränderlichen Sauerstoffverbrauchs erlaubt wiederum Rückschlüsse auf den lokalen Energieverbrauch, der mit dem Sauerstoffverbrauch direkt korreliert ist. Besonders interessant ist, dass dies wiederum Rückschlüsse auf den Glukosespiegel zulässt. Somit erlaubt die erfindungsgemäße Messvorrichtung vorteilhafterweise auch eine nicht-invasive Bestimmung des Blutglukosespiegels.

Die zwei Strahlungsquellen der optischen Messeinheit der erfindungsgemäßen Messvorrichtung sollten so ausgelegt sein, dass die von diesen jeweils bestrahlten Volumenbereiche hinsichtlich der Durchblutung mit sauerstoffarmem bzw. sauerstoffreichem Blut unterschiedlich betroffen sind. Dies kann z. B. dadurch erreicht werden, dass die wenigstens zwei Strahlungsquellen unterschiedliche räumliche Abstrahlcharakteristiken haben. So können als Strahlungsquellen z. B. eine Leuchtdiode und ein Laser verwendet werden, die ähnliche Wellenlängen (z. B. 630 nm und 650 nm) haben. Die beiden Strahlungsquellen unterscheiden sich aber durch den Öffnungswinkel der Abstrahlung. Während z. B. die Leuchtdiode unter einem großen Öffnungswinkel in das untersuchte Körpergewebe einstrahlt, tritt das Licht der Laserdiode unter einem sehr kleinen Öffnungswinkel in das Körpergewebe ein. Dies hat zur Folge, dass mit den beiden Strahlungsquellen unterschiedliche Volumenbereiche des Körpergewebes erfasst werden. Aufgrund des großen Öffnungswinkels wird von der Leuchtdiode ein größerer Volumenbereich der nicht-durchbluteten Epidermis erfasst als von dem Laser. Die undurchblutete Epidermis ist von einer Änderung der Hämoglobinkonzentration praktisch nicht betroffen. Dementsprechend ist die Intensität der von dem Körpergewebe gestreuten und/oder transmittierten Strahlung der Leuchtdiode weniger stark von einer Änderung der Hämoglobinkonzentration abhängig als die Intensität der Strahlung des Lasers. Voraussetzung ist, dass die Wellenlänge der von den beiden Strahlungsquellen jeweils emittierten Strahlung so gewählt wird, dass die Strahlung unterschiedlich stark durch Oxihämoglobin bzw. Desoxihämoglobin absorbiert wird. Die Wellenlänge sollte daher zwischen 600 und 700 nm, vorzugsweise zwischen 630 und 650 nm liegen.

Die Auswertungseinheit der erfindungsgemäßen Messvorrichtung kann mit Vorteil zur Bestimmung eines lokalen metabolischen Parameters aus der von dem Körpergewebe gestreuten und/oder transmittierten Strahlung der beiden Strahlungsquellen ausgebildet sein. Wenn in dem untersuchten Körpergewebe Sauerstoff verbraucht wird, wird Oxihämoglobin in Desoxihämoglobin umgewandelt. Durch einen Vergleich der aus den unterschiedlichen Volumenbereichen des Körpergewebes stammenden Strahlung der beiden Strahlungsquellen kann die Änderung des Konzentrationsverhältnisses von Oxihämoglobin und Desoxihämoglobin festgestellt werden. Hieraus ergibt sich wiederum der lokale Sauerstoffverbrauch und daraus letztlich (indirekt) der Blutglukosespiegel. Somit ist die Auswertungseinheit der erfindungsgemäßen Messvorrichtung sinnvollerweise eingerichtet zur Bestimmung des lokalen Sauerstoffverbrauchs und/oder des Blutglukosespiegels anhand der Intensitäten der von dem Körpergewebe gestreuten und/oder transmittierten Strahlung der beiden Strahlungsquellen.

Sinnvollerweise sind die Strahlungsquellen und die Strahlungssensoren bei der erfindungsgemäßen Messvorrichtung an der Bedienoberfläche der Tastatur angeordnet, beispielsweise seitlich beabstandet von den Bedientasten der Tastatur. Bei dieser Ausgestaltung kann die optische Messeinheit der Messvorrichtung jederzeit bei der Arbeit am Computer benutzt werden, beispielsweise indem ein Finger auf ein Messfeld an der Bedienoberfläche der Tastatur gelegt wird, in welchem die Strahlungsquellen und die Strahlungssensoren angeordnet sind. Alternativ können die Strahlungsquellen und die Strahlungssensoren an der Außenseite des Gehäuses des betreffenden mobilen Unterhaltung- oder Kommunikationsgerätes angeordnet sein, um eine jederzeitige Nutzung der Messvorrichtung zu ermöglichen. Die Strahlungsquellen und -sensoren müssen nicht direkt an der Oberfläche angeordnet sein. Alternativ kann die Strahlung über optische Fasern übertragen werden, die von der Oberfläche der Tastatur bzw. des Gerätegehäuses zu den im Inneren angeordneten Strahlungsquellen bzw. -sensoren geführt sind.

Gemäß einer bevorzugten Ausgestaltung weist die erfindungsgemäße Messvorrichtung zusätzlich eine in die Tastatur bzw. in das mobile Gerät der Unterhaltungs- oder Kommunikationselektronik integrierte Messeinheit zur Erfassung von lokalen Gewebeparametem, wie Fettgehalt, Wassergehalt und/oder Durchblutung auf, wobei die Auswertungseinheit eingerichtet ist zur Bestimmung des wenigstens einen lokalen metabolischen Parameters aus den Signalen der optischen Messeinheit und den Gewebeparametern.

Ein wichtiger lokaler Gewebeparameter im Sinne der Erfindung ist beispielsweise die Durchblutung. Damit sind die durchblutungsbedingten Volumenschwankungen des untersuchten Körpergewebes gemeint. Zur Erfassung der Durchblutung kann die erfindungsgemäße Messvorrichtung insofern, wie oben bereits erwähnt, mit einer Plethysmographieeinheit herkömmlicher Art (z. B. Photoplethysmograph) ausgestattet sein. Somit kann die optische Messeinheit der erfindungsgemäßen Messvorrichtung gleichzeitig zur Erfassung der lokalen Gewebeparameter genutzt werden.

Die Erfindung basiert u. a. auf der Erkenntnis, dass durch die Kombination der Erfassung von oximetrischen und plethysmographischen Signalen die Möglichkeit eröffnet wird, lokale metabolische Parameter zu bestimmen.

Für die Ermittlung des lokalen Sauerstoffverbrauchs sollte mittels der erfindungsgemäßen Messvorrichtung zusätzlich zur oximetrisch bestimmten arteriellen Sauerstoffkonzentration auch die kapillare Sauerstoffkonzentration im Gewebe bestimmt werden können. Hierzu muss allerdings die Zusammensetzung des untersuchten Körpergewebes bekannt sein. Entscheidende Parameter sind der lokale Fettgehalt und/oder der Wassergehalt des Körpergewebes. Diese Parameter können beispielsweise mittels bioelektrischer Impedanzmessung erfasst werden. Gemäß einer sinnvollen Ausgestaltung der Erfindung ist also eine herkömmliche (optische) Oximetrieeinheit mit einer bioelektrischen Impedanzmesseinheit in einem einzigen Gerät kombiniert. Aus den mittels der bioelektrischen Impedanzmesseinheit gewonnenen Messsignalen kann die Zusammensetzung des untersuchten Körpergewebes bestimmt werden. Auf dieser Grundlage kann dann aus den oximetrischen Signalen mittels der Auswertungseinheit der Messvorrichtung die kapillare Sauerstoffsättigung im Gewebe ermittelt werden. Für die bioelektrische Impedanzmessung sind zweckmäßigerweise Elektroden in die Bedienoberfläche der Tastatur bzw. in die Gehäuseoberfläche des Gerätes der Unterhaltungs- oder Kommunikationstechnik integriert. Besonders sinnvoll ist es, wenn jeweils eine Strom einspeisende Elektrode und eine Messelektrode derart an den Außenseiten des Gehäuses z.B. eines Mobiltelefons angeordnet sind, dass der Benutzer des Gerätes mit einer Hand die eine Elektrode und mit der anderen Hand die andere Elektrode berühren kann. Es sollten die gegebenenfalls vorhandenen weiteren Sensoren der erfindungsgemäßen Messvorrichtung ebenfalls im Bereich der Messelektroden angeordnet sein, damit der Benutzer das Gerät mit beiden Händen festhalten kann, während sämtliche erforderliche Messungen gleichzeitig erfolgen. Die Messergebnisse können dann auf dem integrierten Display des Gerätes für den Benutzer gut ablesbar angezeigt werden.

Bei herkömmlichen Messverfahren zur Bestimmung der bioelektrischen Impedanz wird beispielsweise eine Messung zwischen einer Hand und einem Fuß der zu untersuchenden Person durchgeführt, woraus sich ein Globalindex für die jeweils vermessene Körperseite ergibt. Die diagnostische Sensoreinheit der erfindungsgemäßen Messvorrichtung kann eine bioelektrische Impedanzmesseinheit umfassen, wobei diese ein Paar Einspeiseelektroden zur Einspeisung von elektrischem Wechselstrom und ein Paar Messelektroden zur Impedanzmessung aufweist. Der Abstand zwischen dem Einspeiseelektrodenpaar und dem Messelektrodenpaar beträgt sinnvollerweise weniger als 10 cm, vorzugsweise weniger als 1 cm. Durch die Verringerung des Elektrodenabstandes auf wenige Millimeter bis einige Zentimeter wird gemäß der Erfindung nicht über den gesamten Körper integriert, sondern die bioelektrische Impedanz lokal aufgenommen. Dadurch ist es möglich, lokale Änderungen der Impedanz zu bestimmen. Z.B. ändert sich die lokale Bioimpedanz aufgrund der sich ändernden Blutmenge innerhalb eines Pulsschlages. Dies ermöglicht eine Bestimmung der Herzfrequenz über die lokale bioelektrische Impedanz. Als wichtiger physiologischer Parameter wird dabei gleichzeitig die Pulsamplitude bestimmt. Es hat sich gezeigt, dass diese Pulsamplitude wiederum mit der Körpertemperatur korreliert, d.h. es ist möglich, mit Hilfe der Bioimpedanzanalyse die Temperatur der untersuchten Körperstelle zu ermitteln. Weiterhin hängt die lokale Bioimpedanz von der Menge der Flüssigkeit, d.h. von der lokalen Blutmenge im untersuchten Gewebe ab, womit es möglich ist, die lokale Durchblutung des untersuchten Gewebes zu bestimmen. Schließlich ändert sich die lokale bioelektrische Impedanz des Körpers in Abhängigkeit von der Nahrungsaufnahme, so dass mit der Bioimpedanz der Metabolismus untersucht werden kann, welcher entscheidenden Einfluss auf den Blutglukosespiegel besitzt. Die erfindungsgemäße Messvorrichtung ermöglicht somit eine indirekte nicht-invasive Überwachung des Blutglukosewertes, bei der die Wirkung der Glukose bzw. der Energiebedarf der durch Glukose initiierten physiologischen Reaktionen im Körper untersucht wird. Hierzu werden verschiedene physiologische Parameter wie z.B. die Herzaktivität und/oder die Durchblutung und/oder die Körpertemperatur und/oder die Bioimpedanz zur Beschreibung des Metabolismus benutzt. Durch einen geeigneten Algorithmus ist es möglich, aus den aufgenommen Messsignalen der bioelektrischen Impedanzanalyse, ggf. zusammengenommen mit den Messsignalen weiterer Messmodalitäten der erfindungsgemäßen Vorrichtung, den Blutglukosespiegel zu bestimmen.

Eine sinnvolle Weiterbildung der erfindungsgemäßen Messvorrichtung sieht vor, dass die bioelektrische Impedanzmesseinheit außerdem zur Erfassung von globalen Gewebeparametern, wie globaler Fettgehalt und/oder globaler Wassergehalt, ausgebildet ist. Hierdurch wird die Funktionalität der erfindungsgemäßen Messvorrichtung erweitert. Die bioelektrische Impedanzmesseinheit der erfindungsgemäßen Messvorrichtung kann derart ausgestaltet sein, dass damit sowohl lokale wie auch globale Gewebeparameter gemessen werden können.

Die Zusammensetzung des Körpergewebes kann mit der erfindungsgemäßen Messvorrichtung auch optisch bestimmt werden. Das Prinzip der optischen Bestimmung des Körperfettgehalts mittels Infrarotlicht ist aus dem Stand der Technik bekannt. Hierzu kann die optische Messeinheit der erfindungsgemäßen Messvorrichtung genutzt werden.

Gemäß einer vorteilhaften Ausgestaltung umfasst die erfindungsgemäße Vorrichtung einen integrierten Temperatur- oder Wärmesensor. Dieser kann zur Bestimmung der lokalen Wärmeproduktion genutzt werden. Im einfachsten Fall ist der Temperatursensor zur Messung der Oberflächentemperatur der Haut am Messort ausgebildet. Vorzugsweise ist mittels des Wärmesensors eine orts-, zeit- und tiefenaufgelöste Wärmemessung am Messort möglich. Anhand des Wärmeaustauschs kann auf die lokale Stoffwechselaktivität zurückgeschlossen werden. Außerdem ist der Wärmesensor zur Bestimmung der lokalen Durchblutung geeignet. Bezüglich näherer Hintergrundinformationen zur Wärmemessung wird auf die Veröffentlichung von Nitzan et al. verwiesen (Meir Nitzan, Boris Khanokh, "Infrared Radiometry of Thermally Insulated Skin for the Assessment of Skin Blood Flow", Optical Engineering 33, 1994, No. 9, S. 2953 bis 2956). Insgesamt liefert der Wärmesensor Daten, die mit Vorteil zur Bestimmung von metabolischen Parametern genutzt werden können.

Die arterielle Sauerstoffsättigung ($SaO_2$) und die venöse Sauerstoffsättigung ($SvO_2$) bestimmen abhängig von der Art des untersuchten Gewebes die kapillare (arteriovenöse) Sauerstoffsättigung ($StO_2$). Es gilt:

$$K * SvO_2 + (1 - K) * SaO_2 = StO_2,$$

wobei K ein gewebeabhängiger Korrekturfaktor ist, der vom Volumenverhältnis von Arterien zu Venen im untersuchten Gewebe abhängt. Im Mittel liegt dieser Wert etwas unter 0,5. Der für das jeweilige Gewebe maßgebliche Wert kann gemäß der Erfindung durch bioelektrische Impedanzmessung ermittelt werden, um dann aus der obigen Formel die venöse Sauerstoffsättigung zu bestimmen. Mittels Temperatur- bzw. Wärmemessung und/oder bioelektrischer Impedanz (Impedanzplethysmographie) kann die Durchblutung V, d. h. die durchblutungsbedingte Volumenschwankung des Gewebes bestimmt werden. Nach der Beziehung

$$VO_2 = V * (SaO_2 - SvO_2)$$

kann dann schließlich der lokale Sauerstoffverbrauch $VO_2$ berechnet werden, der ein Maß für die metabolische Aktivität am Messort darstellt.

Durch eine EKG-Einheit zur Erfassung eines EKG-Signals über zwei oder mehr EKG-Elektroden wird der Funktionsumfang der erfindungsgemäßen Messvorrichtung vorteilhaft erweitert. Gemäß dieser vorteilhaften Weiterbildung der Erfindung werden mittels der Messvorrichtung plethysmographische Signale und EKG-Signale kombiniert erfasst und ausgewertet. Die Auswertungseinheit der Messvorrichtung kann dann mit Vorteil zur Auswertung des zeitlichen Verlaufs der Volumenpulssignale und der EKG-Signale eingerichtet sein. Mittels einer geeigneten Programmsteuerung ist die Auswertungseinheit der erfindungsgemäßen Messvorrichtung dazu in der Lage, die R-Zacken in dem EKG-Signal automatisch zu erkennen. Damit wird automatisch der exakte Zeitpunkt des Herzschlags ermittelt. Weiterhin ist die Auswertungseinheit aufgrund ihrer Programmsteuerung dazu in der Lage, die Maxima in dem Volumenpulssignal zu erkennen. Anhand der Maxima in dem Volumenpulssignal ist der Zeitpunkt des Eintreffens einer bei einem Herzschlag ausgelösten Pulswelle an dem von der Messvorrichtung erfassten peripheren Messort feststellbar. Somit kann schließlich der zeitliche Abstand zwischen einer R-Zacke in dem EKG-Signal und dem darauf folgenden Maximum in dem Volumenpulssignal ermittelt werden. Dieser zeitliche Abstand ist ein Maß für die so genannte Pulswellengeschwindigkeit. Auf der Basis der Pulswellengeschwindigkeit kann einerseits eine Aussage über den Blutdruck getroffen werden. Eine Verkürzung der Pulswellengeschwindigkeit geht nämlich mit einer Erhöhung des Blutdrucks einher, während eine Verlängerung der Pulswellengeschwindigkeit auf eine Blutdruckerniedrigung schließen lässt. Eine exakte Bestimmung des Blutdrucks aus der Pulswellengeschwindigkeit ist allerdings nicht möglich, es können nur Tendenzen angegeben werden. Weiterhin ist die Pulswellengeschwindigkeit von der Dichte des Blutes und insbesondere von der Elastizität der Blutgefäßwandungen (beispielsweise der Aorta) abhängig. Aus der Elastizität der Blutgefäße kann wiederum auf eine ggf. vorliegende Arteriosklerose geschlossen werden. Es können in diese Auswertung auch die Absolutwerte der Herzfrequenz, die Herzfrequenzvariabilität und entsprechende Arrhythmien des Herzens einbezogen werden. So können automatisch Arrhythmien wie Sinus Tachycardia, Sinus Bradycardia, Sinus Arrest und so genannte Escape Beats festgestellt werden. Anhand des EKG-Signals können außerdem Aussagen über die zeitliche Dauer der Vorhofkontraktion des Herzens bei einem Herzschlag, die zeitliche Dauer der Herzkammerkontraktion sowie die Dauer der Relaxation der Herzkammer usw. festgestellt werden. Außerdem sind Vordiagnosen bezüglich so genannter Blocks in der Leitung der elektrischen Erregungssignale am Herzen (AV-Block, Bundle Branch-Block usw.) und auch bezüglich Durchblutungsstörungen oder Infarkten möglich. Weitere Irregularitäten im Pulsverlauf sind anhand des Volumenpulssignals feststellbar.

Durch die Kombination der Auswertung des EKG-Signals und des Volumenpulssignals bei der automatischen Auswertung ist die erfindungsgemäße Messvorrichtung zur funktionalen Bewertung des Gefäßsystems des Patienten selbsttätig in der Lage. Auf der Grundlage der automatisch ausgewerteten Signale kann die erfindungsgemäße Vorrichtung den (globalen) kardiovaskulären Zustand oder allgemein die Fitness des Benutzers grob einschätzen und bei Anzeichen einer Arteriosklerose oder sonstiger Herz-Kreislauf-Probleme ein entsprechendes Warnsignal oder einen leicht interpretierbaren Fitness- oder Risikoindikator für den Benutzer der Vorrichtung erzeugen und diesen, beispielsweise über einen an den Computer, mit dem die Tastatur verbunden ist, angeschlossenen Monitor oder über ein integriertes Display des mobilen Gerätes der Unterhaltungs- oder Kommunikationstechnik anzeigen. Somit kann die erfindungsgemäße Messvorrichtung vorteilhaft zur Selbstdiagnose von Herz-Kreislauf-Erkrankungen verwendet werden.

Besonders vorteilhaft ist die erfindungsgemäße Kombination der vorgenannten Messverfahren, nämlich der Oximetrie, der EKG-Messung, der Temperatur- bzw. Wärmemessung und ggf. der bioelektrischen Impedanzmessung. Mittels der Auswertungseinheit der Vorrichtung können sämtliche Messsignale durch einen geeigneten Algorithmus ausgewertet und kombiniert werden, um den Metabolismus zu untersuchen. Durch die Kombination der verschiedenen Messmodalitäten wird eine hohe Effektivität und Zuverlässigkeit bei der Erkennung von pathologischen Veränderungen erreicht. Sämtliche Parameter können mit Vorteil zu einem globalen Index zusammengefasst werden, der für den Benutzer leicht interpretierbar ist und ihm einen direkten und fundierten Hinweis auf seinen allgemeinen Gesundheitszustand gibt.

Die Kombination der verschiedenen Messmodalitäten, die in der erfindungsgemäßen Messvorrichtung, wie oben beschrieben, zusammengefasst sein können, ist weiterhin vorteilhaft, weil dadurch, wie oben bereits erwähnt, eine nicht-invasive indirekte Messung der Glukosekonzentration möglich ist. Ein mögliches Vorgehen bei der Bestimmung des Blutglukosespiegels mittels der erfindungsgemäßen Vorrichtung wird im Folgenden näher erläutert:

Die erfindungsgemäße Messvorrichtung dient zur Messung und zur Auswertung von Daten, die durch den Stoffwechsel beeinflusst werden. Es leuchtet unmittelbar ein, dass dabei der Energiehaushalt und die Zusammensetzung der von einem Benutzer der Messvorrichtung aufgenommenen Nahrung eine große Rolle spielen. Die Nährstoffe, die am Stoffwechsel beteiligt sind, sind bekanntlich im Wesentlichen Kohlenhydrate, Fette und Eiweiße. Kohlenhydrate werden zur weiteren Verarbeitung in Glukose, Eiweiße in Aminosäuren, und Fette in Fettsäuren umgewandelt. Die Energieträger werden dann wiederum in den Zellen des Körpergewebes zusammen mit Sauerstoff unter Abgabe von Energie zu ATP (Adenosintriphosphorsäure) umgewandelt. ATP ist der eigentliche körpereigene Energieträger. Die Verwendung von Glukose zur Erzeugung von ATP ist bevorzugt. Wenn die Erzeugung von ATP aus Glukose jedoch (z. B. wegen eines Mangels an Insulin) gehemmt ist, findet stattdessen eine verstärkte Fettsäure-Oxidation statt. Der Sauerstoffverbrauch ist bei diesem Prozess allerdings ein anderer.

Die Reaktion des Metabolismus des menschlichen Körpers auf eine Nahrungsaufnahme hängt, wie zuvor erwähnt, von der Zusammensetzung der Nahrung charakteristisch ab. So reagiert beispielsweise das vaskuläre System des Körpers in Abhängigkeit davon, wie viel Energie der Körper zur Verdauung der aufgenommenen Speisen benötigt. Anhand der mittels der erfindungsgemäßen Messvorrichtung bestimmbaren Pulswellengeschwindigkeit sowie auch anhand der Blutdruckamplitude und des Pulses lässt sich die Reaktion des Körpers auf die Nahrungsaufnahme bestimmen. Hierzu ist zweckmäßigerweise die Auswertungseinheit der erfindungsgemäßen Messvorrichtung zur Auswertung des zeitlichen Verlaufs der Pulswellengeschwindigkeit und zur Ermittlung der Zusammensetzung von einem Benutzer der Messvorrichtung aufgenommener Nahrung anhand des zeitlichen Verlaufs der Pulswellengeschwindigkeit ab dem Zeitpunkt der Nahrungsaufnahme eingerichtet. Die Pulswellengeschwindigkeit, sowie auch die Blutdruckamplitude und der Puls ändern sich, sobald die Nahrungsaufnahme beginnt. Die Maxima und die jeweiligen Zeitpunkte der Maxima sind dabei beeinflusst durch die Nahrungszusammensetzung. Der Verlauf und die absolute Höhe von Pulswellengeschwindigkeit, Blutdruckamplitude und Puls können herangezogen werden, um mittels der Auswertungseinheit der erfindungsgemäßen Messvorrichtung die Zusammensetzung der aufgenommenen Nahrung zu bestimmen.

Der Metabolismus des menschlichen Körpers ist im Normalzustand, d. h. in Ruhe und in der so genannten thermoneutralen Zone, im Wesentlichen durch den Glukosehaushalt bestimmt. Daher kann die Glukosekonzentration in den Zellen des Körpergewebes in diesen Normalzustand als reine Funktion der Wärmeproduktion und des Sauerstoffverbrauchs beschrieben werden. Es gilt:

$$[Glu] = f_1(\Delta T, VO_2) \,,$$

wobei [Glu] für die Glukosekonzentration steht. Die Wärmeproduktion $\Delta T$ kann mittels des Wärmesensors der erfindungsgemäßen Messvorrichtung z.B. aus der Differenz zwischen der arteriellen Temperatur und der Temperatur, welche die Hautoberfläche bei perfekter thermischer Isolierung erreichen würde, bestimmt werden ($\Delta T = T_\infty - T_{Arterie}$). $f_1(\Delta T, VO_2)$ gibt die funktionale Abhängigkeit der Glukosekonzentration von der Wärmeproduktion und vom Sauerstoffverbrauch an. Der Sauerstoffverbrauch ergibt sich, wie oben beschrieben, aus dem Unterschied zwischen venöser und arterieller Sauerstoffsättigung und der Durchblutung. Zur Bestimmung der Glukosekonzentration während bzw. direkt nach der Nahrungsaufnahme muss jedoch ein Korrekturterm berücksichtigt werden, der den Anteil des Fettstoffwechsels am Energiehaushalt wiedergibt. Es gilt dann:

$$[Glu] = f_1(\Delta T, VO_2) + X \cdot f_2(\Delta T, VO_2)$$

X ist ein Faktor, der nach der Nahrungsaufnahme negativ ist. Dabei hängt X von der Zusammensetzung der aufgenommenen Nahrung ab. Insbesondere ist X davon abhängig, in welchem Verhältnis Fett und Kohlenhydrate am Metabolismus beteiligt sind. Der Faktor X lässt sich, wie oben beschrieben, anhand des zeitlichen Verlaufs der Pulswellengeschwindigkeit bestimmen. X ist 0, wenn reine Kohlenhydrate oder direkt Glukose aufgenommen werden. Der Betrag von X steigt an, je größer der Anteil von Fett an der aufgenommenen Nahrung ist. Zur Bestimmung des Korrekturfaktors X aus dem zeitlichen Verlauf der Pulswellengeschwindigkeit, der Blutdruckamplitude und/oder des Pulses wird normalerweise eine Eichung der erfindungsgemäßen Messvorrichtung zur Anpassung an den jeweiligen Benutzer der Vorrichtung erforderlich sein. $f_2(\Delta T, VO_2)$ gibt für den Fettstoffwechsel die funktionale Abhängigkeit der Glukosekonzentration von der Wärmeproduktion und vom Sauerstoffverbrauch an.

Die Auswertungseinheit der erfindungsgemäßen Messvorrichtung kann somit zur Bestimmung der lokalen Glukosekonzentration aus dem lokalen Sauerstoffverbrauch und der lokalen Wärmeproduktion eingerichtet sein. Hierzu muss die Messvorrichtung die geeigneten Messmodalitäten aufweisen. Die Ermittlung des Sauerstoffverbrauchs, kann, wie oben erläutert, durch die Kombination der Oximetrie mit der bioelektrischen Impedanzmessung erfolgen. Zur Ermittlung der Wärmeproduktion ist dann noch zusätzlich ein geeigneter Wärmesensor erforderlich. Um schließlich die Glukosekonzentration nach dem oben angegebenen funktionalen Zusammenhang berechnen zu können, sollte noch der Korrekturfaktor X, beispielsweise aus dem zeitlichen Verlauf der Pulswellengeschwindigkeit, ermittelt werden. Dies kann, wie ebenfalls oben erläutert, durch kombinierte Messung von EKG-Signalen und plethysmographischen Signalen erfolgen. Zur Bestimmung der Glukosekonzentration sind also zweckmäßigerweise in der erfindungsgemäßen Messvorrichtung ein Pulsoximeter, eine EKG-Einheit, eine bioelektrische impedanzmesseinheit sowie ein Wärmesensor kombiniert.

Die zuvor skizzierte Methode erlaubt zunächst nur eine Bestimmung der intrazellulären Glukosekonzentration. Mit der Blutglukosekonzentration besteht vereinfacht der folgende Zusammenhang:

$$[Glu]_{Zelle} = a + b * \ln (c * [Glu]_{Blut})$$

Die Konstanten a, b und c hängen von der individuellen Physiologie des Benutzers der Messvorrichtung ab. Somit kann die Auswertungseinheit der erfindungsgemäßen Messvorrichtung weiterhin eingerichtet sein zur Bestimmung des Blutglukosespiegels aus der lokalen Glukosekonzentration, wobei von der Physiologie des Benutzers der Messvorrichtung abhängige Parameter berücksichtigt werden müssen. Diese Parameter können durch entsprechende Eichung bestimmt werden, beispielsweise durch Vergleich mit in herkömmlicher Weise invasiv bestimmten Blutglukosewerten.

Für die praktische Realisierung der erfindungsgemäßen Messvorrichtung bildet vorteilhafterweise der Computer, an den die Tastatur angeschlossen ist, die Auswertungseinheit, wobei die oben beschriebenen Funktionen der Auswertungseinheit durch auf dem Computer ablaufende Software realisiert sind und wobei die mittels der Software ermittelten physiologischen Parameter mittels des Computers gespeichert werden. Bei dieser Ausgestaltung wird die bei dem Computer ohnehin vorhandene Datenverarbeitungselektronik zur Verarbeitung der mittels der diagnostischen Messeinheit gewonnen Messsignale benutzt. Dies lässt sich durch Bereitstellung entsprechender Software leicht bewerkstelligen. Gleichzeitig können die mittels der Software ermittelten physiologischen Parameter mittels des Computers gespeichert werden. Dies ermöglicht es, den Verlauf einer Erkrankung und die Effekte einer entsprechenden Therapie zu verfolgen und zu dokumentieren.

Alternativ dient als Auswertungseinheit ein in dem Gerät der Unterhaltungs- oder Kommunikationstechnik ohnehin vorhandener integrierter Mikroprozessor oder -controller, wobei die Funktionen der Auswertungseinheit durch auf dem Mikroprozessor oder -controller ablaufende Software realisiert sind.

Sinnvoll ist es weiterhin, wenn die erfindungsgemäße Vorrichtung eine Diagnoseeinheit zur Bewertung der mittels der Auswertungseinheit ermittelten physiologischen Parameter aufweist. Die Funktionen der Diagnoseeinheit können wiederum durch Software realisiert sein. Die Auswertungseinheit ist dafür zuständig, die erfassten Signale auszuwerten, um daraus die für die Diagnostik erforderlichen Parameter in der oben beschriebenen Art und Weise zu bestimmen. Diese Parameter werden dann von der Diagnoseeinheit weiter verarbeitet, um daraus Rückschlüsse bezüglich etwaiger Erkrankungen zu ziehen. Die Diagnoseeinheit ist auch dafür zuständig, insbesondere bei Verwendung der Messvorrichtung zur Selbstdiagnose durch einen Benutzer, das Vorliegen einer Erkrankung automatisch zu erkennen und gegebenenfalls ein entsprechendes Warnsignal für den Benutzer zu erzeugen.

Sinnvollerweise ist also die Diagnoseeinheit der erfindungsgemäßen Messvorrichtung zur Bestimmung des Status des Herz-Kreislauf-Systems aus den mittels der Auswertungseinheit ermittelten Parametern eingerichtet. Gemäß einer besonders vorteilhaften Ausgestaltung der Erfindung ist die Diagnoseeinheit außerdem zur Berechnung eines globalen Fitnessindex auf der Basis des Status des Herz-Kreislauf-Systems und den (mittels bioelektrischer Impedanzmessung erfassten) globalen Gewebeparametem eingerichtet. Somit können die globalen Gewebeparameter genutzt werden, um den globalen Fitnessindex zu erhalten, der besonders aufschlussreich Auskunft über den momentanen Gesundheitszustand des Benutzers gibt. Zur Bestimmung des globalen Fitnessindex können sämtliche erfassten Messwerte des Benutzers einbezogen werden. Gegebenenfalls wird eine Mittelung über einen vorgebbaren Zeitraum durchgeführt. Neben den kardiovaskulären Messwerten und den globalen Gewebeparametem (globaler Fettgehalt, globaler Wassergehalt) können auch die lokalen Gewebeparameter sowie die lokalen metabolischen Parameter (z. B. lokaler Sauerstoffverbrauch) mit berücksichtigt werden. Das Ergebnis ist dann der globale Fitnessindex als einzelner Wert, der für den Benutzer der Messvorrichtung besonders einfach interpretierbar ist.

Zweckmäßigerweise ist mit dem Computer der erfindungsgemäßen Messvorrichtung eine Anzeigeeinheit, beispielsweise ein herkömmlicher Monitor, zur Anzeige der ermittelten physiologischen Parameter und zur Ausgabe der mittels der Diagnoseeinheit erzeugten Untersuchungsergebnisse verbunden. Zur Anzeige eignet sich alternativ ein bei einem Gerät der Unterhaltungs- und Kommunikationstechnik sowieso vorhandenes Display.

Sinnvollerweise ist die erfindungsgemäße Messvorrichtung eingerichtet zur Datenfernübertragung, nämlich zur Übertragung der medizinischen Messsignale oder der ermittelten physiologischen Parameter über ein Daten- oder Kommunikationsnetz. Die Datenübertragung kann z.B. mittels des Computers, an den die Tastatur mit der Messeinheit gemäß der Erfindung angeschlossen ist, über ein Datennetz (z.B. Internet) erfolgen. Alternativ können die physiologischen Parameter über ein Mobilfunknetz übermittelt werden, wenn die Messeinheit gemäß der Erfindung z.B. in ein Mobiltelefon integriert ist. Die Messsignale oder die physiologischen Parameter können zum Beispiel an eine zentrale Stelle ("Healthcare-Center") zur eingehenderen Analyse und Dokumentation sowie zur Überwachung der zeitlichen Entwicklung einzelner Parameter übermittelt werden. Dort werden die Daten z.B. mittels geeigneter Analysealgorithmen ggf. unter Berücksichtung von hinterlegten Patientendaten (einschließlich Informationen bezüglich chronischer Erkrankungen oder Vorerkrankungen) ausgewertet. Das Ergebnis kann wiederum über das jeweilige Daten- oder Kommunikationsnetz an die Messvorrichtung zurück gesendet werden, um den Benutzer der Vorrichtung entsprechend über seinen Gesundheitszustand zu informieren. Von der zentralen Stelle aus können auch bei Bedarf weitere gezielte Messungen mittels der erfindungsgemäßen Messvorrichtung initiiert werden. Außerdem können zum Zwecke einer erweiterten Anamnese veranlasst durch die Auswertungsergebnisse Rückfragen an den Patienten über das Daten- oder Kommunikationsnetz übermittelt werden. Die Daten und Auswertungsergebnisse können automatisch an einen behandelnden Arzt übermittelt werden. Falls sich aus den Mess- und Auswertungsergebnissen Hinweise auf einen medizinischen Notfall ergeben, können die erforderlichen Maßnahmen (z.B. automatische Alarmierung des Rettungsdienstes) sofort in die Wege geleitet werden. Ein weiterer Vorteil der Datenfernübertragung ist, dass die erforderliche Software zur Auswertung der Messsignale nicht in der Vorrichtung selbst implementiert sein muss, sondern lediglich an der zentralen Stelle, wo die Daten empfangen werden, vorgehalten und gepflegt werden muss.

Eine besonders praktische Ausgestaltung der erfindungsgemäßen Messvorrichtung ergibt sich, wenn der Computer ein mobiles Gerät, insbesondere ein Notebook, ein Laptop, ein Palmtop oder ein Handheld ist. In diesem Falle können die diagnostischen Messungen von dem Benutzer des Computers jederzeit, auch unterwegs, durchgeführt werden.

Bei photoplethysmographischen Messungen hat der Anpressdruck des Fingers auf den optischen Sensor signifikanten Einfluss auf die Messsignale. Demzufolge kann es sinnvoll sein, die erfindungsgemäße Messvorrichtung mit Mitteln zur Bestimmung des Anpressdrucks eines auf die Messeinheit aufgelegten Fingers auszustatten. Es kann sich dabei um herkömmliche Drucksensoren, beispielsweise in Form eines piezoresistiven Elements handeln. Ebenso möglich sind optische Verfahren zur Bestimmung des Fingeranpressdrucks. Denkbar ist es auch, den Fingeranpressdruck aus den (photoplethysmographischen) Signalen selbst zu bestimmen, da sich der Fingeranpressdruck charakteristisch auf die Messsignale auswirkt. Der ermittelte Fingeranpressdruck kann dann bei der weiteren Auswertung der Messsignale berücksichtigt werden, um den Einfluss des Anpressdrucks beispielsweise auf die Durchblutung zu kompensieren.

Eine sinnvolle Weiterbildung der erfindungsgemäßen Messvorrichtung ergibt sich durch Integration eines zusätzlichen Temperatursensors zur Bestimmung der Raumtemperatur in der Umgebung der Messvorrichtung. Es bietet sich beispielsweise die Verwendung eines Temperatursensors an, dessen Funktion auf der Messung der Schallausbreitungsgeschwindigkeit in der Umgebungsluft basiert. Die Umgebungstemperatur beeinflusst den Metabolismus des menschlichen Körpers. Insofern ist es sinnvoll, die Umgebungstemperatur bei der Auswertung der mittels der Messvorrichtung gewonnenen Messsignale zu berücksichtigen.

Gemäß einer vorteilhaften Ausgestaltung der Erfindung weist die Messvorrichtung einen oder mehrere Temperatursensoren zur Bestimmung der Körperkerntemperatur auf. Sinnvollerweise sind die Temperatursensoren in einer Position angeordnet, in welche sie direkt mit den zu untersuchenden Körperstellen in Kontakt gebracht werden können. Alternativ kann der Temperatursensor zur Bestimmung der Körperkerntemperatur eine von der Computertastatur oder von dem mobilen Gerät separate Einheit sein, die über eine drahtlose (z.B. Bluetooth) oder drahtgebundene Verbindung mit der Tastatur bzw. mit dem mobilen Gerät verbunden ist. Auf diese Weise kann die Einheit zur Messung der Körperkerntemperatur problemlos gehandhabt werden, um die Temperatur zu messen. Es kann eine übliche Sensorik an sich bekannter Art zur Messung der Körperkerntemperatur verwendet werden. Die Körperkerntemperatur ist eine wichtige Bezugsgröße, die bei der Bestimmung physiologischer und insbesondere metabolischer Parameter im Sinne der Erfindung einbezogen werden kann.

Die Auswertungseinheit der erfindungsgemäßen Messvorrichtung kann vorteilhafterweise eingerichtet sein zur Bestimmung der Atemfrequenz aus den Messsignalen der Messeinheit. So kann die Atemfrequenz beispielsweise anhand der photoplethysmographisch ermittelten Pulsamplitude abgeschätzt werden. Die Pulsamplitude hängt wiederum reziprok von der Herzfrequenz ab.

Gemäß einer bevorzugten Ausgestaltung der Erfindung ist vorgesehen, dass die wenigstens eine diagnostische Sensoreinheit eine von dem mobilen Gerät der Unterhaltungs- oder Kommunikationstechnik separate Einheit bildet, die mit dem mobilen Gerät über eine schnurlose oder schnurgebundene Signalleitung verbunden ist. Das bedeutet, dass zwar die Funktionalität der Messvorrichtung in das mobile Gerät integriert ist. Die Sensorik selbst ist allerdings von dem mobilen Gerät getrennt. Diese Ausgestaltung eignet sich insbesondere zur kontinuierlichen Überwachung des Blutglukosewertes oder anderer physiologischer Parameter, da die Messeinheit z.B. unabhängig von dem mobilen Gerät dauerhaft an einer geeigneten Stelle des Körpers des Benutzers platziert werden kann. Die Auswertungseinheit kann sich dabei entweder ebenfalls in der Messeinheit oder in dem mobilen Gerät, mit dem die Messeinheit verbunden ist, befinden.

Die erfindungsgemäße Messvorrichtung kann in ihrer Funktionalität dadurch noch erweitert werden, dass die diagnostische Sensoreinheit eine Einheit zur Untersuchung von Proben, wie Blut-, Urin-, Stuhl-, Atemluft, Schweiß-, Speichel-, Haar-, Hormonproben oder dergleichen, umfaßt. Damit erfüllt die Vorrichtung gleichsam die Funktion eines privaten medizinischen Labors, das vom Benutzer der Vorrichtung selbst einfach zu bedienen ist. Diese Funktion ist ebenso zur kontinuierliche Überwachung wie zur Anwendung im Notfall sinnvoll.

Gemäß einer sinnvollen Weiterbildung der Erfindung sind die optische Messeinheit und der Temperatur- oder Wärmesensor in einem gemeinsamen Sensorgehäuse angeordnet, wobei an der Oberseite des Sensorgehäuses eine flächige EKG-Elektrode ausgebildet ist, die wenigstens eine Ausnehmung für den Durchtritt der von der wenigstens einen Strahlungsquelle emittierten Strahlung aufweist. Die flächige EKG-Elektrode weist sinnvollerweise eine weitere Ausnehmung für den Temperatur- oder Wärmesensor auf. Die Strahlungsquelle, der Strahlungssensor und der Temperatur- oder Wärmesensor können auf einer gemeinsamen Platine innerhalb des Sensorgehäuses angeordnet sein. Somit sind die benötigten Messmodalitäten in dem Sensorgehäuse zusammengefasst, welches eine Einheit bildet, die in eine Computertastatur oder in ein mobile Gerät problemlos und flexibel integrierbar ist. Denkbar ist es auch, das Sensorgehäuse mit einer Tastatur oder einem mobilen Gerät (z.B. über eine geeignete Steckverbindung) lösbar verbindbar auszugestalten, so dass die Messsensorik nur bei Bedarf verfügbar ist. Außerdem ermöglicht diese Ausgestaltung ein nachträgliches Ausrüsten einer Tastatur oder eines mobilen Gerätes mit der erfindungsgemäßen Messfunktionalität. Das Sensorgehäuse sollte Abmessungen von weniger als 1 cm x 1 cm x 1 cm haben, um problemlos und flexibel im Sinne der Erfindung eingesetzt werden zu können. An der Oberseite des Sensorgehäuses kann außerdem wenigstens eine zusätzliche flächige Elektrode ausgebildet sein, die als Einspeise- oder Messelektrode der Impedanzmesseinheit dient, um zusätzlich eine bioelektrische Impedanzmessung zu ermöglichen.

Gemäß einer sinnvollen Weiterbildung der Erfindung weist die Messvorrichtung zusätzlich eine Blutdruck-Messeinheit zur Messung des systolischen und/oder diastolischen Blutdrucks auf. Der Blutdruck ist eine wichtige Größe, die bei der Bestimmung von physiologischen und metabolischen Parametern gemäß der Erfindung zusätzlich mit einbezogen werden kann, um die Zuverlässigkeit der Ergebnisse zu verbessern. Die Blutdruck-Messeinheit kann eine Blutdruckmanschette üblicher Art mit zugehöriger Sensorik sein, wie sie bei heute handelsüblichen Blutdruckmessgeräten eingesetzt werden, z.B. bei elektronischen Blutdruckmessgeräten, die am Handgelenk benutzt werden. Die Blutdruck-Messeinheit kann drahtgebunden oder drahtlos mit der Computertastatur bzw. mit dem mobilen Gerät gemäß der Erfindung verbunden sein.

Ausführungsbeispiele der Erfindung werden im Folgenden unter Bezugnahme auf die Zeichnungen näher erläutert. Es zeigen:

Figur 1    schematische Ansicht eines ersten Ausführungsbeispiels der erfindungsgemäßen Messvorrichtung;

Figur 2    Darstellung der erfindungsgemäßen Vorrichtung anhand eines Blockdiagramms;

Figur 3    Blockdiagramm-Darstellung der Oximetrieeinheit der erfindungsgemäßen Messvorrichtung;

Figur 4    Blockdiagramm-Darstellung der Wärmemesseinheit;

Figur 5    Blockdiagramm-Darstellung der Impedanzmesseinheit der Messvorrichtung;

Figur 6    Blockdiagramm-Darstellung der EKG-Einheit der Messvorrichtung;

Figur 7    schematische Ansicht eines zweiten Ausführungsbeispiels der erfindungsgemäßen Messvorrichtung;

Figur 8    Illustration der lokalen bioelektrischen Impedanzmessung gemäß der Erfindung;

Figur 9    Illustration der diagnostischen Sensoreinheit der erfindungsgemäßen Messvorrichtung.

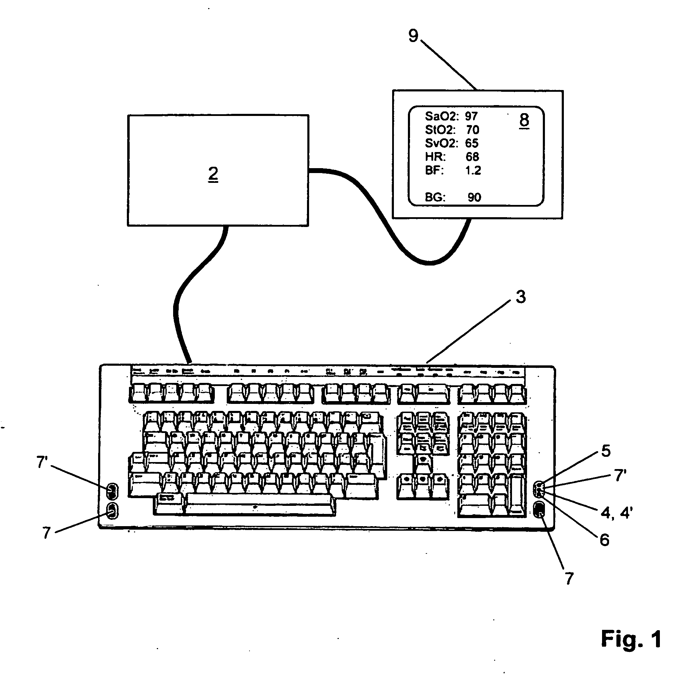

Die Figur 1 zeigt ein erstes Ausführungsbeispiel der erfindungsgemäßen Messvorrichtung. Die Messvorrichtung umfasst einen Computer 2, der mit einer Tastatur 3 verbunden ist. In die Bedienoberfläche der Tastatur 3 sind verschiedene medizinische Messmodalitäten integriert. Diese berührt der Benutzer der Vorrichtung zur Durchführung einer Messung mit den Fingerspitzen. In die Tastatur sind Lichtquellen 4, 4' beispielsweise in Form von Leuchtdioden integriert, die dazu in der Lage ist, Licht bei verschiedenen Wellenlängen zu emittieren. Hierzu können beispielsweise verschiedene lichtemittierende Halbleiterelemente in einem gemeinsamen Gehäuse untergebracht sein. Ebenso denkbar ist die Verwendung von Lichtwellenleitern, um das Licht von verschiedenen Lichtquellen an die Bedienoberfläche der Tastatur 3 zu führen. Des Weiteren umfasst die Tastatur 3 einen oder mehrere Fotosensoren 5. Die Fotosensoren sind in unmittelbarer Nähe zur Lichtquelle 4 bzw. 4' angeordnet. Die Sensoren 5 empfangen das im Gewebe an der Fingerspitze des Benutzers gestreute Licht der Lichtquelle 4 bzw. 4'. Weiterhin ist unmittelbar neben der Lichtquelle 4 bzw. 4' ein Wärmesensor 6 vorgesehen. Dadurch ist gewährleistet, dass die Bestimmung der Durchblutung anhand der Wärmemessung am selben Messort erfolgt wie die optische Messung. Außerdem sind an der Bedienoberfläche der Tastatur 3 insgesamt vier Elektroden 7 bzw. 7' zur Messung der lokalen bioelektrischen Impedanz vorgesehen. Der Benutzer der Vorrichtung berührt mit einer Hand jeweils zwei Elektroden 7 bzw. 7' gleichzeitig. Eine der beiden Kontaktflächen dient zur Aufprägung eines elektrischen Stromes am Messort, während die andere Kontaktfläche zur Spannungsmessung genutzt wird. Auf diese Weise ist sichergestellt, dass die Messergebnisse nicht von den Kontaktwiderständen der Messelektroden beeinflusst sind. Die beiden mit der Bezugsziffer 7 bezeichneten Elektroden werden außerdem als EKG-Elektroden einer ebenfalls in die Tastatur 3 integrierten EKG-Einheit der Messvorrichtung verwendet. Die beiden Elektroden werden jeweils mit den Fingerspitzen berührt, so dass sich eine Zweipunkt-Ableitung (Arm-zu-Arm-Messung) ergibt. Die mittels der verschiedenen in die Tastatur 3 integrierten Sensoren aufgenommenen Messsignale werden mittels des Computers 2 verarbeitet. Die so gewonnenen physiologischen Parameter werden dann auf einer Anzeigefläche 8 eines an den Computer 2 angeschlossenen Monitors 9 ausgegeben. Angezeigt werden z. B. die arterielle ($SaO_2$), die kapillare ($StO_2$) und die venöse ($SvO_2$) Sauerstoffsättigung. Angezeigt wird weiterhin die ermittelte Herzfrequenz (HR), der Fettgehalt des Gewebes (BF). Schließlich wird noch ein Blutglukosewert (BG) angezeigt. Der Benutzer der Messvorrichtung kann jederzeit die ihn interessierenden physiologischen Parameter ermitteln. Hierzu legt er lediglich die Finger, mit denen er ansonsten die Tasten der Tastatur 3 betätigt, auf die Elektroden 7, 7'. Die Parameter werden dann nach der Verarbeitung der Messsignale mittels des Computers 2 sofort mittels des Monitors 9 angezeigt. Der Benutzer der Vorrichtung 1 muss seine Arbeit am Computer 2 also zur Ermittlung der physiologischen Parameter praktisch nicht unterbrechen.

Bei dem in der Figur 1 dargestellten Ausführungsbeispiel sind zwei Strahlungsquellen 4 und 4' vorgesehen, welche unterschiedliche Volumenbereiche des untersuchten Körpergewebes bestrahlen. Hierzu haben die zwei Strahlungsquellen 4 und 4' unterschiedliche räumliche Abstrahlcharakteristiken, nämlich unterschiedliche Abstrahlwinkel. Bei der Strahlungsquelle 4 handelt es sich um eine Leuchtdiode, während es sich bei der Strahlungsquelle 4' um einen Laser, beispielsweise einen so genannten VCSEL-Laser (engl. "vertical cavity surface emitting laser") handelt. Sowohl die Leuchtdiode 4 als auch der Laser 4' emittieren Licht mit sehr ähnlicher Wellenlänge (z. B. 630 nm und 650 nm), aber mit unterschiedlichen Öffnungswinkeln (z. B. 25° und 55°). Mit der in der Figur 1 dargestellten Anordnung ist - wie oben beschrieben - eine differenzielle Messung von Metabolismus-induzierten Änderungen des Sauerstoffgehalts im Blut möglich. Hierzu muss die Wellenlänge der von den beiden Strahlungsquellen 4 und 4' jeweils emittierten Strahlung in einem Bereich liegen, in welchem das Licht von Oxihämoglobin und Desoxihämoglobin unterschiedlich stark absorbiert wird. Für eine Absolutmessung des Sauerstoffgehalts des Blutes (Sauerstoffsättigung) müssen weitere Strahlungsquellen (in der Figur 1 nicht dargestellt) vorhanden sein, deren Lichtwellenlänge in einem Spektralbereich liegt, in welchem die Lichtabsorption von Oxihämoglobin und Desoxihämoglobin im Wesentlichen gleich ist (so genannter isobektischer Punkt). Das von der Leuchtdiode bzw. von dem Laser emittierte Licht kann mittels entsprechender Lichtleitfasern an die entsprechende Stelle an der Bedienoberfläche der Tastatur geführt werden. In diesem Fall sind mit den Bezugsziffern 4 und 4' in der Figur 1 die entsprechenden Faserenden dargestellt. Es ist möglich, die Leuchtdiode und den Laser so an die entsprechenden Fasern anzukoppeln, dass sie mit dem gewünschten unterschiedlichen Öffnungswinkel in das zu untersuchende Körpergewebe einstrahlen. Dementsprechend werden mit beiden Strahlungsquellen unterschiedliche Volumina des Körpergewebes untersucht. Aufgrund des größeren Öffnungswinkels ist der Anteil der nicht-durchbluteten Epidermis an dem mittels der Leuchtdiode untersuchten Körpergewebe größer als beim Laser. Das im Körpergewebe gestreute und teilweise absorbierte Licht sowohl der Strahlungsquelle 4 als auch der Strahlungsquelle 4' wird mittels der Sensoren 5 detektiert. Die Sensoren 5 müssen nicht direkt an der Bedienoberfläche der Tastatur 3 angeordnet sein. Stattdessen kann das Licht über Lichtleitfasern den im inneren der Tastatur 3 angeordneten Sensoren zugeführt werden. Zur Unterscheidung des Lichtes der Strahlungsquelle 4 von dem Licht der Strahlungsquelle 4' können die beiden Lichtquellen 4 und 4' unterschiedlich zeitlich moduliert betrieben werden, wobei die mittels der Sensoren 5 detektierten Signale entsprechend demoduliert werden. Alternativ ist es möglich, die Strahlung der beiden Strahlungsquellen 4 und 4' anhand der unterschiedlichen Wellenlänge zu unterscheiden. Die Strahlungsintensität der von den Strahlungsquellen 4 und 4' emittierten Strahlung wird mit der Weglänge beim Durchgang durch das Körpergewebe geschwächt, wobei der Zusammenhang der Intensitätsschwächung mit der Konzentration der absorbierenden Substanz (oxigeniertes Hämoglobin) durch das bekannte Lambert-Beersche Gesetz gegeben ist. Mittels der in der Figur 1 dargestellten Sensoren 5 können die interessierenden Parameter der Intensitätsschwächung bestimmt werden, und zwar getrennt für die von den Strahlungsquellen 4 und 4' jeweils erfassten Volumenbereiche des untersuchten Körpergewebes. Die den verschiedenen Strahlungsquellen 4 und 4' zuzuordnenden Parameter der Intensitätsschwächung können mittels der Auswertungseinheit der erfindungsgemäßen Messvorrichtung zueinander in Beziehung gesetzt werden, um auf diese Weise eine differenzielle Messung durchzuführen. Im einfachsten Fall werden aus den Parametern der Intensitätsschwächung der Strahlung der beiden Strahlungsquellen 4 und 4' jeweils Quotienten berechnet. Aus Änderungen dieser Quotienten kann dann auf Änderungen im Metabolismus zurückgeschlossen werden. Steigt beispielsweise nach der Nahrungsaufnahme der Blutglukosespiegel, gelangt (nach einer gewissen zeitlichen Verzögerung) entsprechend mehr Glukose in die Zellen des Körpergewebes und wird dort umgesetzt. Dabei wird Sauerstoff verbraucht. Diesen Sauerstoff erhalten die Zellen über das Blut. Dabei wird aus dem oxigenierten Hämoglobin durch Abgabe von Sauerstoff desoxigeniertes Hämoglobin. Dementsprechend steigt das Verhältnis von desoxigeniertem Hämoglobin zu oxigeniertem an. Aufgrund der unterschiedlichen Öffnungswinkel der Strahlung der Strahlungsquellen 4 und 4' wirken sich die Änderungen der Hämoglobinkonzentration unterschiedlich auf die jeweilige Intensitätsschwächung aus. Somit können aus den Quotienten der Parameter der Intensitätsschwächung Veränderungen der Hämoglobinkonzentration detektiert werden. Dies ermöglicht es, indirekt auf den Sauerstoffverbrauch zurückzuschließen. Da der Sauerstoffverbrauch seinerseits von dem Blutglukosespiegel abhängt, kann mittels der erläuterten differenziellen Messung der Strahlungsabsorption auch der Blutglukosespiegel ermittelt werden. Als sinnvolle Ergänzung wird parallel zur optischen Messung eine Bioimpedanzanalyse durchgeführt, wozu die in der Figur 1 dargestellten Elektroden 7 und 7' vorgesehen sind. Zweck der Bioimpedanzmessung ist vor allem die Bestimmung der lokalen Durchblutung. Diese kann als weiterer Parameter bei der Bestimmung des Sauerstoffverbrauchs und damit auch des Blutglukosespiegels herangezogen werden. Unterschiedliche Öffnungswinkel der Strahlung können auch mit nur einer Strahlungsquellen 4 durch Verwendung entsprechender optischer Elemente (z.B. Strahlteiler, Linsen etc.) erzeugt werden.

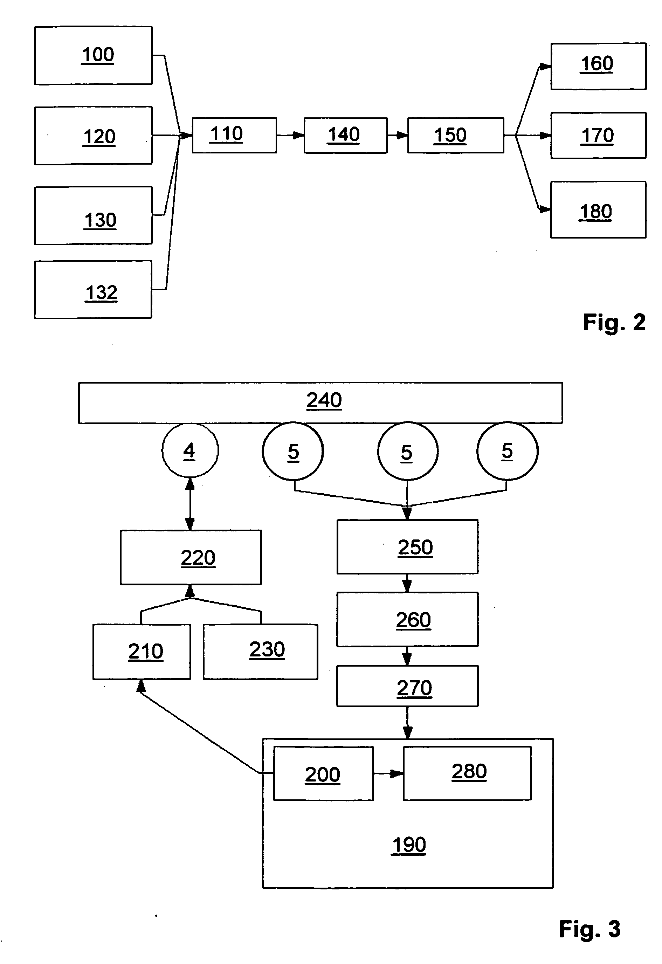

Die Figur 2 zeigt schematisch den Aufbau der erfindungsgemäßen Messvorrichtung als Blockdiagramm. Die Vorrichtung 1 umfasst eine optische Messeinheit 100 zur optischen Messung der Sauerstoffkonzentration im Blutgefäßsystem des Körpergewebes am jeweiligen Messort. Die mittels der optischen Messeinheit 100 erfassten oximetrischen und plethysmographische Signale werden einer Analyseeinheit 110 zugeführt. Eine weitere wesentliche Komponente der Vorrichtung 1 ist eine Wärmemesseinheit 120 zur Bestimmung der lokalen Wärmeproduktion. Bei der Wärmemesseinheit 120 handelt es sich um einen speziellen Wärmesensor, welcher die jeweils untersuchte Körperstelle isoliert. Diese Stelle kann somit nur noch Wärme durch den Blutstrom aufnehmen oder abgeben. Daher ist es möglich, durch die zeitaufgelöste Messung der Temperatur die Durchblutung und die Wärmeproduktion zu bestimmen. Bei einer starken Durchblutung erreicht die untersuchte Körperstelle in sehr kurzer Zeit ihre maximale Temperatur. Bei geringer Durchblutung dauert dies länger. Zusätzlich kann über die Extrapolation der gemessenen Temperatur auf die arterielle Temperatur geschlossen werden, da die Temperatur am Ort der Messung nur durch die arterielle Temperatur und durch die lokale Wärmeproduktion bestimmt wird. Auch die mittels der Wärmemesseinheit 120 erfassten Messsignale werden der Analyseeinheit 110 zur Weiterverarbeitung zugeführt. Außerdem umfasst die Messvorrichtung eine Impedanzmesseinheit 130, die zur Erfassung von lokalen Gewebeparametem mittels bioelektrischer Impedanzmessung dient. Die Messsignale der Impedanzmesseinheit 130 werden ebenfalls mittels der Analyseeinheit 110 verarbeitet. Schließlich ist gemäß der Erfindung noch eine EKG-Einheit 132 zur Erfassung eines EKG-Signals vorgesehen. Auch die EKG-Einheit 132 ist zur Verarbeitung der EKG-Signale mit der Analyseeinheit 110 verbunden. Der optischen Messeinheit 100 sind die Lichtquelle 4 sowie die Lichtsensoren 5 der in der Figur 1 dargestellten Tastatur 3 zugeordnet. Die Wärmemesseinheit 120 ist mit dem Wärmesensor 6 verbunden. Die Impedanzmesseinheit 130 erfasst Messsignale über die Elektroden 7 bzw. 7' der Tastatur 3. Die Analyseeinheit 110 führt eine Vorverarbeitung sämtlicher Messsignale durch. Hierzu durchlaufen die Signale ein Bandpass-Filter, um Störungen im Bereich der Netzfrequenz von 50 bzw. 60 Hz herauszufiltern. Des Weiteren werden die Signale einer Rauschunterdrückung unterzogen. Nach Passieren der Analyseeinheit 110 gelangen die aufbereiteten Signale der optischen Messeinheit 100, der Wärmemesseinheit 120, der Impedanz-Messeinheit 130 und der EKG-Einheit 132 in eine Auswertungseinheit 140. Die Auswertungseinheit 140 ist dafür zuständig, aus den Messsignalen die für die Diagnose wesentlichen Parameter zu berechnen. Die Funktionen der Auswertungseinheit 140 sind im Wesentlichen durch Software realisiert, die auf dem Computer 2 abläuft. Aus den zeitabhängig aufgenommenen Messsignalen der Impedanzmesseinheit 130 wird zunächst die Zusammensetzung des untersuchten Körpergewebes (Wassergehalt, Fettgehalt usw.) berechnet. Aus den Signalen der optischen Messeinheit 100 wird die arterielle Sauerstoffsättigung und - unter Zugrundelegung der zuvor auf der Basis der Impedanzmessung ermittelten Gewebeparameter - die kapillare Sauerstoffsättigung berechnet. Weiterhin werden aus den Messsignalen der Wärmemesseinheit 120 und aus den plethysmographischen Daten, die aus der zeitabhängigen Impedanzmessung ableitbar sind, die Durchblutung und die arterielle Temperatur bestimmt. Aus den Signalen der EKG-Einheit 132 und denjenigen der optischen Messeinheit 100 wird die Pulswellengeschwindigkeit bestimmt. Schließlich werden mittels der Auswertungseinheit 140 aus den Ergebnissen sämtlicher zuvor durchgeführter Berechnungen die venöse Sauerstoffsättigung, und daraus weitere metabolische Parameter, insbesondere der lokale Sauerstoffverbrauch und die Glukosekonzentration am Messort berechnet. Die Berechnungsergebnisse werden mittels einer Diagnoseeinheit 150 interpretiert. Die Diagnoseeinheit 150, die ebenfalls als Software auf dem Computer 2 implementiert ist, dient zur Bewertung der mittels der Auswertungseinheit 140 berechneten lokalen metabolischen Parameter. Die Auswertungseinheit 140 und die Diagnoseeinheit 150 sind zur Anzeige der Messresultate mit einer Grafikeinheit 160 verbunden, die ihrerseits den Monitor 9 ansteuert. Die gewonnenen Daten sind in einer Speichereinheit 170 speicherbar, und zwar unter gleichzeitiger Speicherung des Datums und der Uhrzeit der jeweiligen Messung. Außerdem ist eine Schnittstelleneinheit 180 vorgesehen, die zur Verbindung des Computers 2 mit einem Datennetzwerk zur Übertragung der berechneten physiologischen Parameter dient. Über die Schnittstelleneinheit 180 können sämtliche Daten und Parameter, insbesondere auch die in der Speichereinheit 170 gespeicherten Daten und Parameter, an einen nicht näher dargestellten PC eines behandelnden Arztes übertragen werden. Dort können die Daten detaillierter analysiert werden. Insbesondere können über einen längeren Zeitraum mit der Vorrichtung 1 aufgenommene Daten und Parameter auf Veränderungen hin untersucht werden, um daraus Schlussfolgerungen hinsichtlich der Entwicklung einer bestehenden Erkrankung ableiten zu können.

Die Figur 3 illustriert den Aufbau der optischen Messeinheit 100 der erfindungsgemäßen Vorrichtung 1. Die optische Messeinheit 100 umfasst einen Mikrokontroller 190. Bestandteil des Mikrokontrollers 190 ist ein Timing-Generator 200. Dieser erzeugt Steuerungssignale, die einer Modulationseinheit 210 zugeführt werden. Dadurch wird die zeitliche Modulation der Lichtemission der Leuchtdiode 4 gesteuert. Die Leuchtdiode 4 ist über eine Regelungseinheit 220 mit der Modulationseinheit 210 verbunden. Die Intensität des von der Leuchtdiode 4 emittierten Lichtes ist außerdem über eine Leistungssteuerungseinheit 230 anpassbar. Die Leuchtdiode 4 ist dazu in der Lage, Licht bei zumindest drei verschiedenen Wellenlängen zu emittieren. Hierzu sind verschiedene Licht emittierende Halbleiterbauelemente in einem einzigen Gehäuse der Leuchtdiode 4 vereinigt. Mittels des Timing-Generators 200 wird die zeitliche Abfolge der Lichtemission bei den verschiedenen Lichtwellenlängen gesteuert. Die in den Messkopf 3 der Vorrichtung 1 integrierten Photosensoren 5 sind ebenso wie die Leuchtdiode 4 mit dem in der Figur 3 schematisch angedeuteten Körpergewebe 240 des Benutzers in Kontakt. In dem Körpergewebe 240 wird das Licht der Leuchtdiode 4 gestreut und entsprechend der Sauerstoffkonzentration des Blutes, das das Gewebe 240 durchströmt, absorbiert. Das gestreute Licht wird von den Photosensoren 5 registriert. Der Photostrom jedes Photosensors 5 wird mittels eines Konverters 250 in eine Spannung umgewandelt, mittels eines Verstärkers 260 verstärkt und mittels eines Analog/Digital-Wandlers 270 in digitale Messsignale umgewandelt. Die Digitalsignale werden sodann einem Demodulator 280 zugeführt, der Bestandteil des Mikrokontrollers 190 ist. Der Demodulator 280 separiert die aufgenommenen Messsignale nach den entsprechenden Lichtwellenlängen. Schließlich werden die Signale an die Analyseeinheit 110 weitergegeben.

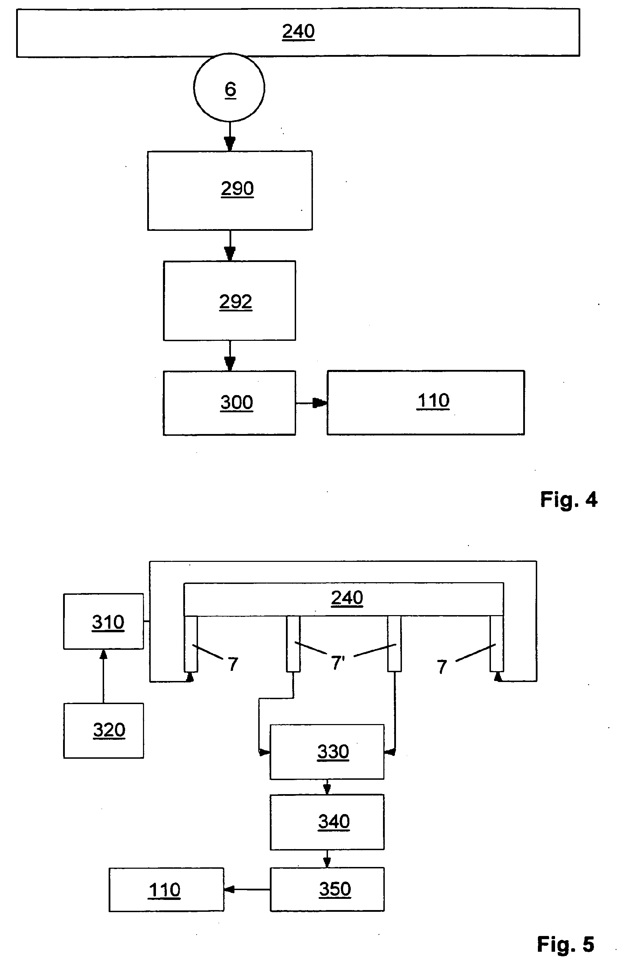

Anhand der Figur 4 wird der Aufbau der Wärmemesseinheit 120 der erfindungsgemäßen Messvorrichtung erläutert. Der Wärmesensor 6, der mit dem Körpergewebe 240 in Berührung ist, weist mehrere nicht näher dargestellte Temperaturmesselemente sowie ein wärmeleitendes Element auf. Sobald der Sensor 6 mit dem Gewebe 240 in Kontakt kommt, beginnt ein Wärmeaustausch. Mittels der Temperaturmesselemente wird die Temperatur an verschiedenen Stellen an dem wärmeleitenden Element des Sensors 6 gemessen. Hieraus kann die in dem Gewebe 240 lokal produzierte Wärme (orts-, zeit- und tiefenaufgelöst) bestimmt werden. Die mittels der Temperaturmesselemente erfassten Signale durchlaufen einen Impedanzwandler 290 sowie einen Verstärker 292 und werden mittels eines Analog/Digital-Wandlers 300 digitalisiert. Die digitalen Messsignale werden sodann der Analyseeinheit 110 zur weiteren Verarbeitung zugeführt. Ein geeigneter Wärmesensor 6 ist beispielsweise in der Veröffentlichung von Ok Kyung Cho et al. (Ok Kyung Cho, Yoon Ok Kim, Hiroshi Mitsumaki, Katsuhiko Kuwa, "Noninvasive Measurement of Glucose by Metabolic Heat Conformation Method", Clinical Chemistry 50, 2004, Nr. 10, S. 1894 bis 1898) beschrieben.

In der Figur 5 ist der Aufbau der Impedanzmesseinheit 130 der erfindungsgemäßen Messvorrichtung dargestellt. Die Impedanzmesseinheit 130 umfasst Elektroden 7 und 7'. Über Kontaktflächen 7 wird dem untersuchten Körpergewebe 240 ein Wechselstrom aufgeprägt, der mittels einer Stromquelle 310 erzeugt wird. Die Stromquelle 310 wird von einem Sinusgenerator 320 angesteuert. Die Frequenz des Wechselstroms variiert zwischen 20 kHz und 100 kHz. Über Kontaktflächen 7' wird eine Spannung als Messsignal am Körpergewebe 240 abgegriffen. Aus dem Verhältnis der gemessenen Spannung zu dem aufgeprägten Strom kann auf die Impedanz des Körpergewebes 240 zurückgeschlossen werden. Hierzu wird die Spannung mittels eines Verstärkers 330 verstärkt und mittels eines Filters 340 gefiltert, um Störsignale zu eliminieren. Wiederum erfolgt eine Digitalisierung mittels eines Analog/Digital-Wandlers 350. Die digitalisierten Messwerte werden wiederum der Analyseeinheit 110 zur weiteren Verarbeitung zugeführt.

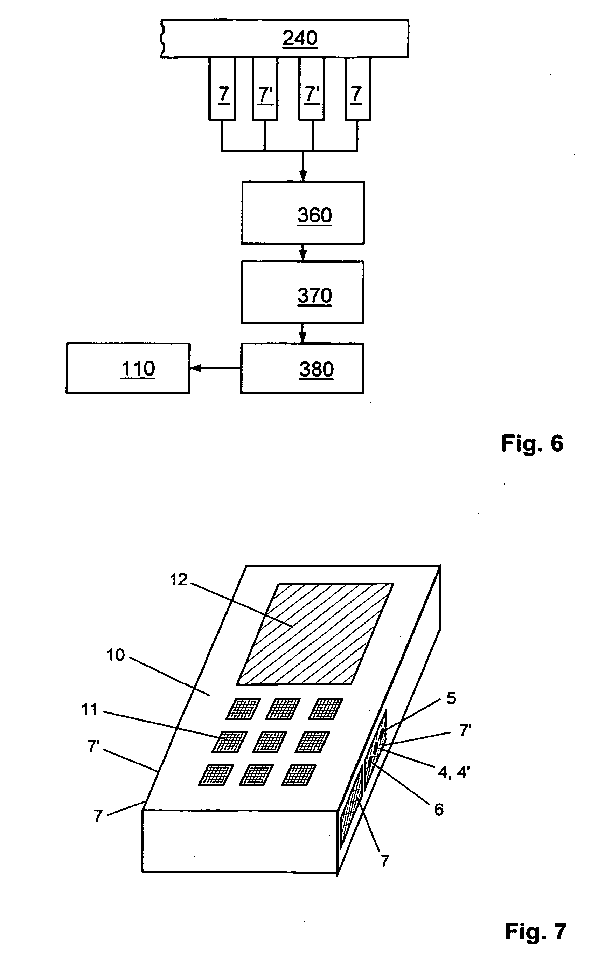

Anhand der Figur 6 wird der Aufbau der EKG-Einheit 132 der erfindungsgemäßen Messvorrichtung veranschaulicht. Die EKG-Einheit 132 erfasst ein EKG-Signal über EKG-Elektroden 7. Es sind dies die Elektroden der Impedanzmesseinheit 130. Die Elektroden 7 haben also bei dem dargestellten Ausführungsbeispiel eine Doppelfunktion. Für eine brauchbare Zweipunkt-Ableitung des EKG-Signals wird dadurch erzeugt, dass, wie oben erwähnt, die beiden Elektroden 7 mit jeweils einer Hand berührt werden. Die beiden Elektroden 7 sind in die Tastatur 3 integriert. Separate, z. B. über Kabel angeschlossene Elektroden sind (für eine einfache Zweipunkt-Ableitung des EKG-Signals) nicht erforderlich. Das abgeleitete EKG-Signal wird mittels Verstärker 360 und Filter 370 aufbereitet. Nach Passieren eines weiteren Analog/Digital-Wandlers 380 wird das Signal an die Analyseeinheit 110 weitergegeben.

Die Figur 7 zeigt ein zweites Ausführungsbeispiel der erfindungsgemäßen Messvorrichtung. Die erfindungsgemäße Messvorrichtung umfasst ein mobiles Gerät 10, nämlich ein Mobiltelefon. An der Vorderseite des Gerätes 10 sind die üblichen Bedientasten 11 zu erkennen. In die seitlichen Flächen des Gehäuses des Gerätes 10 sind verschiedene diagnostische Messsensoren integriert. Diese berührt der Benutzer der Vorrichtung zur Durchführung einer Messung mit den Fingern. In das Gehäuse sind Lichtquellen 4, 4' beispielsweise in Form von Leuchtdioden integriert, die dazu in der Lage ist, Licht bei verschiedenen Wellenlängen zu emittieren. Des Weiteren umfasst das Gerät 10 einen oder mehrere Fotosensoren 5. Die Fotosensoren sind in unmittelbarer Nähe zur Lichtquelle 4 bzw. 4' angeordnet. Die Sensoren 5 empfangen das im Gewebe an der Fingerspitze des Benutzers gestreute Licht der Lichtquelle 4 bzw. 4'. Weiterhin ist unmittelbar neben der Lichtquelle 4 bzw. 4' ein Wärmesensor 6 vorgesehen. Dadurch ist gewährleistet, dass die Bestimmung der Durchblutung anhand der Wärmemessung am selben Messort erfolgt wie die optische Messung. Außerdem sind an den seitlichen Gehäuseoberflächen des Mobiltelefons 10 insgesamt vier Elektroden 7 bzw. 7' zur Messung der lokalen bioelektrischen Impedanz vorgesehen. Der Benutzer des Mobiltelefons 10 berührt mit einer Hand jeweils zwei Elektroden 7 bzw. 7' gleichzeitig. Eine der beiden Kontaktflächen dient zur Aufprägung eines elektrischen Stromes am Messort, während die andere Kontaktfläche zur Spannungsmessung genutzt wird. Auf diese Weise ist sichergestellt, dass die Messergebnisse nicht von den Kontaktwiderständen der Messelektroden beeinflusst sind. Die beiden mit der Bezugsziffer 7 bezeichneten Elektroden werden außerdem als EKG-Elektroden einer ebenfalls in das Mobiltelefon 10 integrierten EKG-Einheit der Messvorrichtung verwendet. Die beiden Elektroden werden jeweils mit den Fingerspitzen berührt, so dass sich eine Zweipunkt-Ableitung (Arm-zu-Arm-Messung) ergibt. Die mittels der verschiedenen in das Mobiltelefon 10 integrierten Sensoren aufgenommenen Messsignale werden mittels des (nicht näher dargestellten) Mikroprozessors des Mobiltelefons 10 verarbeitet. Die so gewonnenen physiologischen Parameter werden dann auf einem Display 12 des Mobiltelefons 10 ausgegeben. Angezeigt werden z. B. die arterielle, die kapillare und die venöse Sauerstoffsättigung. Angezeigt wird weiterhin die ermittelte Herzfrequenz sowie der Fettgehalt des Gewebes. Schließlich wird noch ein Blutglukosewert angezeigt. Der Benutzer der Messvorrichtung kann jederzeit die ihn interessierenden physiologischen Parameter ermitteln. Hierzu legt er lediglich die Finger, mit denen er ansonsten die Tasten 11 betätigt, auf die Elektroden 7, 7'. Die Parameter werden dann nach der Verarbeitung der Messsignale mittels des Mikroprozessors des Mobiltelefons 10 sofort mittels des Displays 12 angezeigt. Die Funktion des erfindungsgemäß als medizinische Messvorrichtung ausgebildeten Mobiltelefons 10 basiert wesentlich auf dem oben beschriebenen indirekten Verfahren zur nicht-invasiven Bestimmung des Blutglukosewertes, bei welchem die Wirkung der Glukose bzw. der Energieumsatz der durch Glukose initiierten physiologischen Reaktionen im Körper untersucht wird. Auf die entsprechende Beschreibung zur Erläuterung des in der Figur 1 gezeigten Ausführungsbeispiels wird verwiesen. Ähnlich wie bei der Tastatur 3 müssen die Lichtquellen 4, 4' und die Sensoren 5 auch bei dem Mobiltelefon 10 nicht direkt an der Gehäuseoberfläche angeordnet sein. Stattdessen kann das Licht über Lichtleitfasern von bzw. zur Gehäuseoberfläche geführt werden, wobei sich die eigentlichen Lichtquellen bzw. Sensoren im Inneren des Gehäuses befinden. Mehrere Lichtquellen bzw. Sensoren können an eine einzige Lichtleitfaser gekoppelt sein.

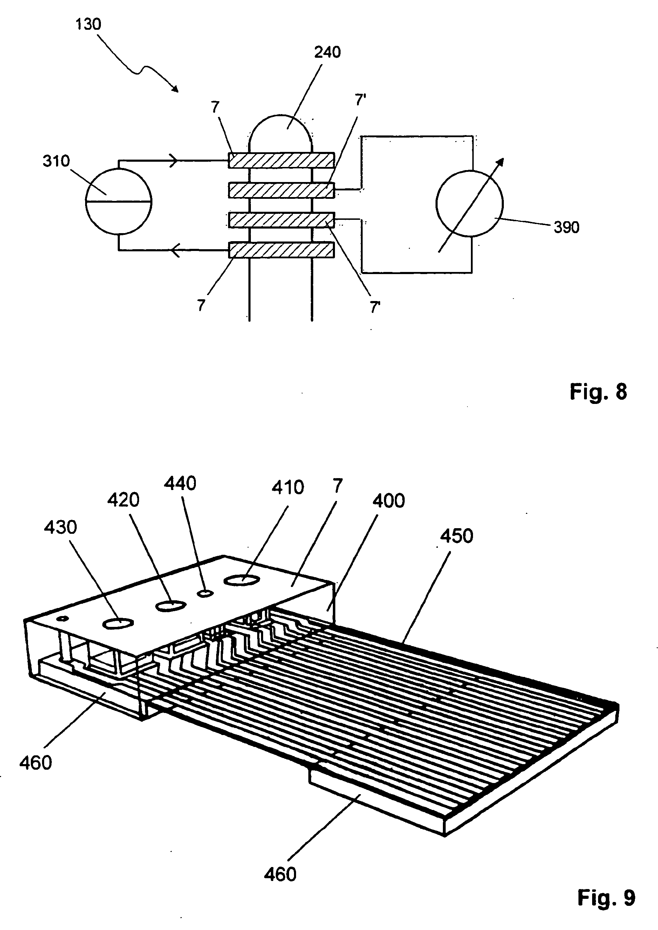

Die in der Figur 8 illustrierte bioelektrische Impedanzmesseinheit 130 der erfindungsgemäßen Messvorrichtung umfasst zur Bestimmung der lokalen Resistanz und der lokalen Reaktanz zwei Elektroden 7 zur Einspeisung von elektrischem Wechselstrom der Stromquelle 310 variabler Frequenz und zwei oder mehr Messelektroden 7' zur Impedanzmessung des Körpergewebes 240 im Bereich des Fingers des Benutzers der Vorrichtung. Aufgrund der Vierpunkt-Messung verfälschen Übergangswiderstände zwischen den Elektroden 7, 7' und dem Körpergewebe 240 die Messung nicht. Sinnvollerweise beträgt der Abstand zwischen den Elektroden 7, 7' nur einige Millimeter bis wenige Zentimeter. Es ist vorteilhaft, wenn mittels der Stromquelle 310 ein Wechselstrom variabler Frequenz erzeugbar ist, da auf diese Weise die Messung der komplexen Impedanz möglich ist. Das Messsignal wird mittels eines Spannungsmessers 390 erfasst. Sinnvollerweise wird das Messsignal mittels eines (in der Figur 8 nicht dargestellten) Analog-/Digitalwandlers digitalisiert und danach einer diskreten Fouriertransformation (DFT) unterzogen. Der DFT-Algorithmus liefert dann den Real- und den Imaginärteil der Impedanz. Die dargestellte Impedanzmesseinheit 130 kann, letztlich aufgrund des geringen Elektrodenabstands, sehr kompakt aufgebaut sein und somit gut in ein mobiles elektronisches Gerät (z.B. Armbanduhr, Mobiltelefon, MP3-Player, Digital-Kamera usw.) integriert werden.

Die Figur 9 illustriert die Konstruktion der diagnostischen Sensoreinheit der erfindungsgemäßen Messvorrichtung. Die verschiedenen Messeinheiten der Sensoreinheit sind in ein Sensorgehäuse 400 mit sehr kleinen äußeren Abmessungen integriert. An der Oberseite des Gehäuses 400 ist eine flächige EKG-Elektrode 7 angeordnet, die aus einer dünnen, elektrisch leitenden Folie besteht. Beim Einbau der Sensoreinheit in eine Computertastatur oder in ein mobiles Gerät wird das Sensorgehäuse 400 so angeordnet, dass der Benutzer die EKG-Elektrode 7 und eine weitere Elektrode (in der Figur 9 nicht dargestellt) zur EKG-Ableitung mit verschiedenen Extremitäten berühren kann. Sinnvollerweise ist die EKG-Elektrode eine dünne Edelstahlfolie. Die kleine Bauform des Mikro-Gehäuses von (bei dem dargestellten Ausführungsbeispiel) 5 mm (B) × 8 mm (L) × 1,8 mm (H) ermöglicht einen flexiblen und kostengünstigen Einbau der Sensoreinheit in unterschiedliche Gehäuse verschiedener am Markt vorhandener Geräte. Zur gleichzeitigen Bestimmung der Sauerstoffsättigung in arteriellem Blut ist in das Sensorgehäuse 400 eine optische Messeinheit, nämlich ein Pulsoximeter integriert. Dieses umfasst zwei oder mehr optische Strahlungsquellen, deren Strahlung durch eine Ausnehmung 410 in der EKG-Elektrode 7 hindurch treten kann. Außerdem umfasst das Pulsoximeter zwei optische Strahlungssensoren, z.B. in Form von Photodioden. Das im Körpergewebe (z.B. eines auf die Elektrode 7 aufgelegten Fingers) gestreute Licht fällt durch zwei Ausnehmungen 420 und 430 in der Elektrode 7 auf die Strahlungssensoren. Die Ausnehmungen 420 und 430 sind in unterschiedlichem Abstand zur Ausnehmung 410 angeordnet. In der Sensoreinheit wird das Licht von zwei oder mehr optischen Strahlungsquellen (z.B. Leuchtdioden) innerhalb des Gehäuses 400 in eine Lichtleitfaser oder in einen geeigneten lichtleitenden Körper gekoppelt, so dass sich an der Oberseite des Mikro-Gehäuses nur eine Ausnehmung 410 für sämtliche Strahlungsquellen befindet und das Licht sämtlicher Strahlungsquellen der Sensoreinheit an derselben Stelle in das zu untersuchende Körpergewebe geleitet wird. Die Photodioden sind jeweils einzeln an eine Lichtleitfaser oder an einen geeignet ausgebildeten lichtleitenden Körper gekoppelt Die optische Messeinheit ermöglicht die gleichzeitige Messung der Sauerstoffsättigung des in dem untersuchten Körpergewebe zirkulierenden Blutes und des Volumenpulses. Sinnvollerweise werden hierfür nicht nur Leuchtdioden, sondern auch andere Strahlungsquellen, wie z.B. Vertical Cavity Surface Emitting Laser (VCSEL), benutzt. Zur gleichzeitigen Bestimmung der thermischen Eigenschaften des untersuchten Gewebes ist in das Sensorgehäuse ein Temperatursensor, nämlich ein Thermistor integriert. Für diesen ist eine weitere Ausnehmung 440 in der EKG-Elektrode 7 vorgesehen. Der Thermistor ist in dem Sensorgehäuse 400 derart angeordnet, dass er guten thermischen Kontakt zum untersuchten Körpergewebe hat. Bei dem dargestellten Ausführungsbeispiel befindet sich der Thermistor zwischen der Ausnehmung 410 für die Lichtleitfaser der optischen Strahlungsquellen und der Ausnehmung 420 für die Lichtleitfaser der ersten Photodiode. Die Sensoreinheit kann problemlos durch eine Impedanzmesseinheit ergänzt werden. Hierzu muss an der Oberseite des Sensorgehäuses 400 wenigstens eine zusätzliche flächige Elektrode (in der Figur 9 nicht dargestellt) ausgebildet sein, die dann als Einspeise- oder Messelektrode der Impedanzmesseinheit dient. Sinnvollerweise können dieselben Messelektroden zur Erfassung des Bioimpedanzsignals und des EKG-Signals benutzt werden. Für den elektrischen Kontakt der Sensoreinheit (z.B. mit der Elektronik eines Mobiltelefons) ist das Sensorgehäuse 400 mit allen integrierten Messeinheiten direkt auf ein Flachbandkabel 450 mit geeigneter Leiterbahnführung montiert, so dass eine einfache elektrische Montage der Sensoreinheit mit Hilfe des Flachbandkabels 450 möglich ist. Das Flachbandkabel 450 kann zu Stabilisierungszwecken an geeigneten Stellen Versteifungen 460 aufweisen.

**Patentansprüche**

1. Messvorrichtung zur nicht-invasiven Bestimmung von wenigstens einem physiologischen Parameter, mit einer diagnostischen Sensoreinheit zur Erzeugung von Messsignalen, und mit einer Auswertungseinheit (140) zur Verarbeitung der Messsignale, wobei die diagnostische Sensoreinheit in die Tastatur (3) eines Computers (2) oder in ein mobiles Gerät (10) der Unterhaltungs- oder Kommunikationstechnik integriert ist, wobei die diagnostische Sensoreinheit umfasst:

   - eine optische Messeinheit (100), die wenigstens eine Strahlungsquelle (4) zur Bestrahlung von zu untersuchendem Körpergewebe (240) und wenigstens einen Strahlungssensor (5) zur Detektion der von dem Körpergewebe (240) gestreuten und/oder transmittierten Strahlung, und
   - eine EKG-Einheit (132) zur Erfassung eines EKG-Signals über zwei oder mehr EKG-Elektroden (7), und
   - einen Temperatur- oder Wärmesensor (6) und
   - eine bioelektrische Impedanzmesseinheit (130),

   **dadurch gekennzeichnet, dass**
   die Auswertungseinheit (140) eingerichtet ist zur Bestimmung des Blutglukosespiegels aus den Messsignalen der optischen Messeinheit (100), der EKG-Einheit (132), des Temperatur- oder Wärmesensors (6) und der bioelektrischen Impedanzmesseinheit (130).

2. Messvorrichtung zur nicht-invasiven Bestimmung von wenigstens einem physiologischen Parameter, mit einer diagnostischen Sensoreinheit zur Erzeugung von Messsignalen, und mit einer Auswertungseinheit (140) zur Verarbeitung der Messsignale, wobei die diagnostische Sensoreinheit in die Tastatur (3) eines Computers (2) oder in ein mobiles Gerät (10) der Unterhaltungs- oder Kommunikationstechnik integriert oder mit diesen verbindbar ist, wobei die diagnostische Sensoreinheit umfasst:

   - eine optische Messeinheit (100), die wenigstens eine Strahlungsquelle (4) zur Bestrahlung von zu untersuchendem Körpergewebe (240) und wenigstens einen Strahlungssensor (5) zur Detektion der von dem Körpergewebe (240) gestreuten und/oder transmittierten Strahlung, und
   - eine EKG-Einheit (132) zur Erfassung eines EKG-Signals über zwei oder mehr EKG-Elektroden (7), und
   - einen Temperatur- oder Wärmesensor (6) und
   - eine bioelektrische Impedanzmesseinheit (130),

   **dadurch gekennzeichnet, dass**
   die Auswertungseinheit (140) eingerichtet ist zur Bestimmung des Blutglukosespiegels aus den Messsignalen der optischen Messeinheit (100), der EKG-Einheit (132), des Temperatur- oder Wärmesensors (6) und der bioelektrischen Impedanzmesseinheit (130), wobei die optische Messeinheit (100) in einem Sensorgehäuse (400) angeordnet sind, wobei an der Oberseite des Sensorgehäuses (400) eine flächige EKG-Elektrode (7) ausgebildet ist, die wenigstens eine Ausnehmung (410) für den Durchtritt der von der wenigstens einen Strahlungsquelle (4) emittierten Strahlung aufweist.

**3.** Messvorrichtung nach Anspruch 2, **dadurch gekennzeichnet, dass** die flächige EKG-Elektrode (7) eine weitere Ausnehmung (440) für den Temperatur- oder Wärmesensor (6) aufweist, wobei die Strahlungsquelle (4), der Strahlungssensor (5) und der Temperatur- oder Wärmesensor (6) auf einer gemeinsamen Platine innerhalb des Sensorgehäuses (400) angeordnet sind.

**4.** Messvorrichtung nach Anspruch 2 oder 3, **dadurch gekennzeichnet, dass** das Sensorgehäuse (400) Abmessungen von weniger als 1 cm x 1 cm x 1 cm hat.

**5.** Messvorrichtung nach einem der Ansprüche 2 bis 4, **dadurch gekennzeichnet, dass** an der Oberseite des Sensorgehäuses (400) wenigstens eine zusätzliche flächige Elektrode ausgebildet ist, die als Einspeise- oder Messelektrode der Impedanzmesseinheit (130) dient.

**6.** Messvorrichtung nach einem der Ansprüche 1 bis 5, **dadurch gekennzeichnet, dass** wenigstens zwei Strahlungsquellen (4, 4') vorgesehen sind, welche unterschiedliche Volumenbereiche des untersuchten Körpergewebes (240) bestrahlen.

**7.** Messvorrichtung nach Anspruch 6, **dadurch gekennzeichnet, dass** die wenigstens zwei Strahlungsquellen (4, 4') unterschiedliche räumliche Abstrahlcharakteristiken haben.

**8.** Messvorrichtung nach einem der Ansprüche 1 bis 7, **dadurch gekennzeichnet, dass** die Auswertungseinheit (140) eingerichtet ist zur Bestimmung des lokalen Sauerstoffverbrauchs und/oder des Blutglukosespiegels anhand der Intensitäten der von dem Körpergewebe (240) gestreuten und/oder transmittierten Strahlung.

**9.** Messvorrichung nach einem der Ansprüche 1 bis 8, **dadurch gekennzeichnet, dass** die biolektrische Impedanzmesseinheit (130) ein Paar Einspeiseelektroden zur Einspeisung von elektrischem Wechselstrom und ein Paar Messelektroden zur Impedanzmessung aufweist, wobei der Abstand zwischen dem Einspeiseelektrodenpaar und dem Messelektrodenpaar weniger als 10 cm, vorzugsweise weniger als 1 cm beträgt.

**10.** Messvorrichtung nach einem der Ansprüche 1 bis 9, **dadurch gekennzeichnet, dass** der Computer (2) die Auswertungseinheit (140) bildet, wobei die Funktionen der Auswertungseinheit (140) durch auf dem Computer (2) ablaufende Software realisiert sind.

**11.** Messvorrichtung nach einem der Ansprüche 1 bis 9, **dadurch gekennzeichnet, dass** ein in das Gerät (10) der Unterhaltungs- oder Kommunikationstechnik integrierter Mikroprozessor oder -controller die Auswertungseinheit (140) bildet, wobei die Funktionen der Auswertungseinheit (140) durch auf dem Mikroprozessor oder -controller ablaufende Software realisiert sind.

**12.** Messvorrichtung nach einem der Ansprüche 1 bis 11, **dadurch gekennzeichnet, dass** sie eine Diagnoseeinheit (150) zur Bewertung der mittels der Auswertungseinheit (140) ermittelten physiologischen Parameter aufweist, wobei die Funktionen der Diagnoseeinheit (150) durch Software realisiert sind.

**13.** Messvorrichtung nach einem der Ansprüche 1 bis 12, **gekennzeichnet durch** eine mit dem Computer (2) verbundene Anzeigeeinheit (9) zur Anzeige der ermittelten physiologischen Parameter.

**14.** Messvorrichtung nach einem der Ansprüche 1 bis 13, **dadurch gekennzeichnet, dass** der Computer (2) zur Übertragung der Messsignale oder des wenigstens einen physiologischen Parameters über ein Daten- oder Kommunikationsnetz eingerichtet ist.

**15.** Messvorrichtung nach einem der Ansprüche 1 bis 14, **dadurch gekennzeichnet, dass** das Gerät (10) der Unterhaltungs- oder Kommunikationstechnik zur Übertragung der Messsignale oder des wenigstens einen physiologischen Parameters über ein Daten- oder Kommunikationsnetz eingerichtet ist.

**16.** Messvorrichtung nach einem der Ansprüche 1 bis 15, **dadurch gekennzeichnet, dass** der Computer (2) ein mobiles Gerät, insbesondere ein Notebook, ein Laptop, ein Palmtop oder ein Handheld ist.

**17.** Messvorrichtung nach einem der Ansprüche 1 bis 16, **gekennzeichnet durch** einen Temperatursensor zur Bestimmung der Raumtemperatur in der Umgebung der Messvorrichtung.

18. Messvorrichtung nach einem der Ansprüche 1 bis 17, **dadurch gekennzeichnet, dass** die Auswertungseinheit (140) eingerichtet ist zur Bestimmung der Atemfrequenz aus den Messsignalen der diagnostischen Messeinheit.

19. Messvorrichtung nach einem der Ansprüche 1 bis 18, **dadurch gekennzeichnet, dass** die diagnostische Sensoreinheit eine von dem mobilen Gerät (10) der Unterhaltungs- oder Kommunikationstechnik separate Einheit bildet, die mit dem mobilen Gerät (10) über eine schnurlose oder schnurgebundene Signalleitung verbunden ist.

20. Messvorrichtung nach einem der Ansprüche 1 bis 19, **dadurch gekennzeichnet, dass** die wenigstens eine diagnostische Sensoreinheit eine Einheit zur Untersuchung von Proben, wie Blut-, Urin-, Stuhl-, Atemluft, Schweiß-, Speichel-, Haar-, Hormonproben oder dergleichen, umfasst.

21. Messvorrichtung nach einem der Ansprüche 1 bis 20, **gekennzeichnet durch** eine Blutdruck-Messeinheit zur Messung des systolischen und/oder diastolischen Blutdrucks.

22. Messvorrichtung nach einem der Ansprüche 1 bis 21, **gekennzeichnet durch** einen weiteren Temperatursensor zur Bestimmung der Körperkerntemperatur.

23. Messvorrichtung nach einem der Ansprüche 1 bis 22, **gekennzeichnet durch** Mittel zur Bestimmung des Anpressdrucks eines auf die optische Messeinheit (100) aufgelegten Fingers.

**Claims**

1. A measuring apparatus for non-invasive determination of at least one physiological parameter comprising a diagnostic sensor unit for producing measurement signals, and an evaluation unit (140) for processing the measurement signals, wherein the diagnostic sensor unit is integrated in the keyboard (3) of a computer (2) or in a mobile device (10) of entertainment or communication technology, wherein the diagnostic sensor unit includes:

   - an optical measuring unit (100) which has at least one radiation source (4) for irradiating body tissue (240) to be examined and at least one radiation sensor (5) for detection of the radiation transmitted and/or scattered by the body tissue (240), and
   - an ECG unit (132) for acquiring an ECG signal by way of two or more ECG electrodes (7), and
   - a temperature and/or heat sensor (6), and
   - a bioelectrical impedance measuring unit (130),

   **characterised in that**
   the evaluation unit (140) is adapted to determine the blood glucose level from the measurement signals of the optical measuring unit (100), the ECG unit (132), the temperature or heat sensor (6) and the bioelectrical impedance measuring unit (130).

2. A measuring apparatus for non-invasive determination of at least one physiological parameter comprising a diagnostic sensor unit for producing measurement signals, and an evaluation unit (140) for processing the measurement signals, wherein the diagnostic sensor unit is integrated in the keyboard (3) of a computer (2) or in a mobile device (10) of entertainment or communication technology or can be connected thereto, wherein the diagnostic sensor unit includes:

   - an optical measuring unit (100) which has at least one radiation source (4) for irradiating body tissue (240) to be examined and at least one radiation sensor (5) for detection of the radiation transmitted and/or scattered by the body tissue (240), and
   - an ECG unit (132) for acquiring an ECG signal by way of two or more ECG electrodes (7), and
   - a temperature and/or heat sensor (6), and
   - a bioelectrical impedance measuring unit (130),

   **characterised in that**
   the evaluation unit (140) is adapted to determine the blood glucose level from the measurement signals of the optical measuring unit (100), the ECG unit (132), the temperature or heat sensor (6) and the bioelectrical impedance measuring unit (130), wherein the optical measuring unit (100) is arranged in a sensor housing (400), wherein provided at the top side of the sensor housing (400) is a planar ECG electrode (7) which has at least one opening (410) for the passage of the radiation emitted by the at least one radiation source (4).

3. A measuring apparatus according to claim 2 **characterised in that** the planar ECG electrode (7) has a further opening (440) for the temperature or heat sensor (6), wherein the radiation source (4), the radiation sensor (5) and the temperature or heat sensor (6) are arranged on a common circuit board within the sensor housing (400).

4. A measuring apparatus according to claim 2 or claim 3 **characterised in that** the sensor housing (4) is of dimensions of less than 1 cm x 1 cm x 1 cm.

5. A measuring apparatus according to one of claims 2 to 4 **characterised in that** provided at the top side of the sensor housing (400) is at least one additional planar electrode serving as a feed-in or measuring electrode of the impedance measuring unit (130).

6. A measuring apparatus according to one of claims 1 to 5 **characterised in that** there are provided at least two radiation sources (4, 4') which irradiate different volume regions of the body tissue (240) being examined.

7. A measuring apparatus according to claim 6 **characterised in that** the at least two radiation sources (4, 4') have different spatial radiation emission characteristics.

8. A measuring apparatus according to one of claims 1 to 7 **characterised in that** the evaluation unit (140) is adapted to determine the local oxygen consumption and/or the blood glucose level on the basis of the intensities of the radiation transmitted and/or scattered by the body tissue (240).

9. A measuring apparatus according to one of claims 1 to 8 **characterised in that** the bioelectrical impedance measuring unit (130) has a pair of feed-in electrodes for feeding in electrical alternating current and a pair of measuring electrodes for impedance measurement, wherein the spacing between the pair of feed-in electrodes and the pair of measuring electrodes is less than 10 cm, preferably less than 1 cm.

10. A measuring apparatus according to one of claims 1 to 9 **characterised in that** the computer (2) forms the evaluation unit (140), wherein the functions of the evaluation unit (140) are implemented by software running on the computer (2).

11. A measuring apparatus according to one of claims 1 to 9 **characterised in that** a microprocessor or microcontroller integrated into the device (10) of entertainment or communication technology forms the evaluation unit (140), wherein the functions of the evaluation unit (140) are implemented by software running on the microprocessor or microcontroller.

12. A measuring apparatus according to one of claims 1 to 11 **characterised in that** it has a diagnostic unit (150) for assessment of the physiological parameters ascertained by means of the evaluation unit (140), wherein the functions of the diagnostic unit (150) are implemented by software.

13. A measuring apparatus according to one of claims 1 to claim 12 **characterised by** a display unit (9) connected to the computer (2) for displaying the ascertained physiological parameters.

14. A measuring apparatus according to one of claims 1 to 13 **characterised in that** the computer (2) is adapted for the transmission of the measurement signals or the at least one physiological parameter by way of a data or communication network.

15. A measuring apparatus according to one of claims 1 to 14 **characterised in that** the device (10) of entertainment or communication technology is adapted for the transmission of the measurement signals or the at least one physiological parameter by way of a data or communication network.

16. A measuring apparatus according to one of claims 1 to 15 **characterised in that** the computer (2) is a mobile device, in particular a notebook, a laptop, a palmtop or a handheld device.

17. A measuring apparatus according to one of claims 1 to 16 **characterised by** a temperature sensor for determining the ambient temperature in the environment of the measuring apparatus.

18. A measuring apparatus according to one of claims 1 to 17 **characterised in that** the evaluation unit (140) is adapted to determine the respiration frequency from the measurement signals of the diagnostic measuring unit.

19. A measuring apparatus according to one of claims 1 to 18 **characterised in that** the diagnostic sensor unit forms a unit separate from the mobile device (10) of entertainment or communication technology and connected to the mobile device (10) by way of a cordless or cord-connected signal line.

20. A measuring apparatus according to one of claims 1 to 19 **characterised in that** the at least one diagnostic sensor unit includes a unit for investigating samples like blood, urine, stools, respiration air, perspiration, saliva, hair, hormone samples or the like.

21. A measuring apparatus according to one of claims 1 to 20 **characterised by** a blood pressure measuring unit for measuring the systolic and/or diastolic blood pressure.

22. A measuring apparatus according to one of claims 1 to 21 **characterised by** a further temperature sensor for determining the body core temperature.

23. A measuring apparatus according to one of claims 1 to 22 **characterised by** means for determining the pressing pressure of a finger applied to the optical measuring unit (100).

**Revendications**

1. Dispositif de mesure pour la détermination non-invasive d'au moins un paramètre physiologique, avec une unité de capteur diagnostique pour la production de signaux de mesure, et avec une unité d'évaluation (140) pour le traitement des signaux de mesure, où l'unité de capteur diagnostique est intégrée dans le clavier (3) d'un ordinateur (2) ou dans un appareil mobile (10) de la technique d'entretien ou de communication, où l'unité de capteur diagnostique comprend:

   - une unité de mesure optique (100) qui comprend au moins une source de rayonnement (4) pour l'irradiation d'un tissu corporel à examiner (240) et au moins un capteur de rayonnement (5) pour la détection du rayonnement dispersé et/ou transmis par le tissu corporel (240), et
   - une unité ECG (132) pour la détection d'un signal ECG par deux ou plusieurs électrodes ECG (7), et
   - un capteur de température ou de chaleur (6) et
   - une unité de mesure d'impédance bioélectrique (130),

   **caractérisé en ce que** l'unité d'évaluation (140) est installée pour la détermination du taux sanguin du glucose à partir des signaux de mesure de l'unité de mesure optique (100), de l'unité ECG (132), du capteur de température ou de chaleur (6) et de l'unité de mesure d'impédance bioélectrique (130).

2. Dispositif de mesure pour la détermination non invasive d'au moins un paramètre physiologique, avec une unité de capteur diagnostique pour la production de signaux de mesure, et avec une unité d'évaluation (140) pour le traitement des signaux de mesure, où l'unité de capteur diagnostique est intégrée dans le clavier (3) d'un ordinateur (2) ou dans un appareil mobile (10) de la technique d'entretien ou de communication ou peut être relié à ceux-ci, ou l'unité de capteur diagnostique comprend:

   - une unité de mesure optique (100) qui comporte au moins une source de rayonnement (4) pour l'irradiation d'un tissu corporel à examiner (240) et au moins un capteur de rayonnement (5) pour la détection du rayonnement dispersé et/ou transmis par le tissu corporel (240), et
   - une unité ECG (132) pour la détection d'un signal ECG par deux ou plusieurs électrodes ECG (7), et
   - un capteur de température ou de chaleur (6) et
   - une unité de mesure d'impédance bioélectrique (130),

   **caractérisé en ce que** l'unité d'évaluation (140) est installée pour la détermination du taux sanguin du glucose à partir des signaux de mesure de l'unité de mesure optique (100), de l'unité ECG (132), du capteur de température ou de chaleur (6) et de l'unité de mesure d'impédance bioélectrique (130), où l'unité de mesure optique (100) sont disposées dans un boîtier de capteur (400), où est réalisé au côté supérieur du boîtier de capteur (400) une électrode ECG plane (7) qui présente au moins un évidement (410) pour le passage du au moins un rayonnement émis par la source de rayonnement (4).

**3.** Dispositif de mesure selon la revendication 2, **caractérisé en ce que** l'électrode ECG plane (7) présente un autre évidement (440) pour le capteur de température ou de chaleur (6), où la source de rayonnement (4), le capteur de rayonnement (5) et le capteur de température ou de chaleur (6) sont disposés sur une platine commune à l'intérieur du boîtier de capteur (400).

**4.** Dispositif de mesure selon la revendication 2 ou 3, **caractérisé en ce que** le boîtier de capteur (400) présente des dimensions inférieures à 1 cm x 1 cm x 1 cm.

**5.** Dispositif de mesure selon l'une des revendications 2 à 4, **caractérisé en ce qu'**est réalisée au côté supérieur du boîtier de capteur (400) au moins une électrode plane additionnelle qui sert d'électrode d'alimentation ou de mesure de l'unité de mesure d'impédance (130).

**6.** Dispositif de mesure selon l'une des revendications 1 à 5, **caractérisé en ce qu'**au moins deux sources de rayonnement (4, 4') sont prévues qui irradient des zones volumiques différentes du tissu corporel examiné (240).

**7.** Dispositif de mesure selon la revendication 6, **caractérisé en ce que** les au moins deux sources de rayonnement (4, 4') ont des caractéristiques de rayonnement spatiales différentes.

**8.** Dispositif de mesure selon l'une des revendications 1 à 7, **caractérisé en ce que** l'unité d'évaluation (140) est installée pour la détermination de la consommation d'oxygène locale et/ou du taux sanguin du glucose à l'aide des intensités du rayonnement dispersé et/ou transmis par le tissu corporel (240).

**9.** Dispositif de mesure selon l'une des revendications 1 à 8, **caractérisé en ce que** l'unité de mesure d'impédance bioélectrique (130) présente une paire d'électrodes d'alimentation pour l'alimentation en courant alternatif électrique et une paire d'électrodes de mesure pour la mesure de l'impédance, l'écart entre la paire d'électrodes d'alimentation et la paire d'électrodes de mesure étant inférieur à 10 cm, de préférence inférieur à 1 cm.

**10.** Dispositif de mesure selon l'une des revendications 1 à 9, **caractérisé en ce que** l'ordinateur (2) constitue l'unité d'évaluation (140), où les fonctions de l'unité d'évaluation (140) sont réalisées par un logiciel exécuté sur l'ordinateur (2).

**11.** Dispositif de mesure selon l'une des revendications 1 à 9, **caractérisé en ce qu'**un microprocesseur ou microcontrôleur intégré dans l'appareil (10) de la technique d'entretien ou de communication constitue l'unité d'évaluation (140), les fonctions de l'unité d'évaluation (140) étant réalisées par un logiciel exécuté sur le microprocesseur ou microcontrôleur.

**12.** Dispositif de mesure selon l'une des revendications 1 à 11, **caractérisé en ce qu'**il présente une unité de diagnostique (150) pour l'évaluation des paramètres physiologiques déterminés par l'unité d'évaluation (140), les fonctions étant réalisées dans l'unité de diagnostique (150) par un logiciel.

**13.** Dispositif de mesure selon l'une des revendications 1 à 12, **caractérisé par** une unité d'affichage (9) reliée à l'ordinateur (2) pour l'affichage des paramètres physiologiques déterminés.

**14.** Dispositif de mesure selon l'une des revendications 1 à 13, **caractérisé en ce que** l'ordinateur (2) est conçu pour la transmission des signaux de mesure ou du au moins un paramètre physiologique par un réseau de données ou de communication.

**15.** Dispositif de mesure selon l'une des revendications 1 à 14, **caractérisé en ce que** l'appareil (10) de la technique d'entretien ou de communication est installé pour la transmission des signaux de mesure ou du au moins un paramètre physiologique par un réseau de données ou de communication.

**16.** Dispositif de mesure selon l'une des revendications 1 à 15, **caractérisé en ce que** l'ordinateur (2) est un appareil mobile, en particulier un ordinateur portable, un ordinateur portatif, un ordinateur de poche ou un organisateur de poche.

**17.** Dispositif de mesure selon l'une des revendications 1 à 16, **caractérisé par** un capteur de température pour déterminer la température ambiante dans l'environnement du dispositif de mesure.

**18.** Dispositif de mesure selon l'une des revendications 1 à 17, **caractérisé en ce que** l'unité d'évaluation (140) est installée pour déterminer la fréquence respiratoire à partir des signaux de mesure de l'unité de mesure diagnostique.

**19.** Dispositif de mesure selon l'une des revendications 1 à 18, **caractérisé en ce que** l'unité de capteur diagnostique forme une unité séparée de l'appareil mobile (10) de la technique d'entretien ou de communication, qui est reliée à l'appareil mobile (10) par une ligne de transmission de signaux sans fil ou à fil.

**20.** Dispositif de mesure selon l'une des revendications 1 à 19, **caractérisé en ce que** la au moins une unité de capteur diagnostique comprend une unité pour examiner des prélèvements comme des prélèvements de sang, d'urine, de selles, d'air respiratoire, de transpiration, de salive, de cheveux, d'hormones ou analogue.

**21.** Dispositif de mesure selon l'une des revendications 1 à 20, **caractérisé par** une unité de mesure de tension artérielle pour la mesure de la tension artérielle systolique et/ou diastolique.

**22.** Dispositif de mesure selon l'une des revendications 1 à 21, **caractérisé par** un capteur de température additionnel pour la détermination de la température corporelle centrale.

**23.** Dispositif de mesure selon l'une des revendications 1 à 22, **caractérisé par** des moyens pour déterminer la pression d'application d'un doigt appliqué à l'unité de mesure optique (100).

SaO2: 97      8
StO2: 70
SvO2: 65
HR:   68
BF:   1.2

BG:   90

**Fig. 1**

**Fig. 2**

**Fig. 3**

240

( 6 )

290

292

300 → 110

**Fig. 4**

240

310

320

7   7'   7

330

340

110 ← 350

**Fig. 5**

24

**Fig. 6**

**Fig. 7**

**Fig. 8**

**Fig. 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0069328 A1
- US 6331162 B
- US 4960126 A
- US 6714814 B
- US 4928014 A
- EP 1407713 A

**In der Beschreibung aufgeführte Nicht-Patentliteratur**

- **MEIR NITZAN ; BORIS KHANOKH.** Infrared Radiometry of Thermally Insulated Skin for the Assessment of Skin Blood Flow. *Optical Engineering,* 1994, vol. 33 (9), 2953-2956
- **OK KYUNG CHO ; YOON OK KIM ; HIROSHI MITSUMAKI ; KATSUHIKO KUWA.** Noninvasive Measurement of Glucose by Metabolic Heat Conformation Method. *Clinical Chemistry,* 2004, vol. 50 (10), 1894-1898